# EUROPÄISCHE PATENTSCHRIFT

(11) **EP 4 404 887 B1**
(45) Veröffentlichungstag und Bekanntmachung des Hinweises auf die Patenterteilung: **04.11.2026**
(21) Anmeldenummer: 22777653.1
(22) Anmeldetag: 22.09.2022
(51) Int. Cl.: A61F 9/008

(54) **ANORDNUNG ZUR REKALIBRIERUNG DES FOKUS EINES OPHTHALMOLOGISCHEN SYSTEMS ZUR INTRAOKULAREN LASERBEHANDLUNG**
ASSEMBLY FOR RECALIBRATING THE FOCUS OF AN OPHTHALMOLOGICAL SYSTEM FOR INTRAOCULAR LASER TREATMENT
ENSEMBLE DE RÉÉTALONNAGE DU FOYER D'UN SYSTÈME OPHTALMOLOGIQUE POUR TRAITEMENT LASER INTRAOCULAIRE

(30) Priorität: 24.09.2021 DE 102021210661
(43) Veröffentlichungstag der Anmeldung: 31.07.2024
(73) Patentinhaber: Carl Zeiss Meditec AG, 07745 Jena (DE)
(72) Erfinder: BUBLITZ, Daniel, 07745 Jena (DE); HACKER, Martin, 07745 Jena (DE); DICK, Manfred, 07926 Gefell (DE); GRATZKE, Alexander, 07745 Jena (DE); MERKEL, Stephan, 07745 Jena (DE); KLOPFLEISCH, Peter, 07751 Jena (DE); HAESE, Jürgen, 07768 Kahla (DE)
(74) Vertreter: Müller, Gabriele
(86) Internationale Anmeldenummer: PCT/EP2022/076402
(87) Internationale Veröffentlichungsnummer: WO 2023/046847

(56) Entgegenhaltungen:
- WO-A1-2015/043771
- WO-A1-2015/054414
- DE-A1- 102019 007 147

## Beschreibung

Die vorliegende Erfindung betrifft eine Anordnung zur Rekalibrierung des Fokus eines ophthalmologischen Laserbehandlungssystems, welches neben einer Behandlungslasereinheit, einer Bildgebungseinheit und einem optischen System zur Fokussierung und Strahlüberlagerung auch über ein OCDR-System und eine Steuereinheit verfügt. Unter OCDR wird hierbei ein eindimensionales OCT verstanden, d.h. welches also A-Scans (Tiefenprofile) aufnehmen kann. Das Bildgebungssystem kann als kamerabasiertes System ausgeführt sein oder auch als Beobachtungseinheit für das Bedienerauge ("Laserspaltlampe").

Nach dem bekannten Stand der Technik existieren bereits zahlreiche Lösungen zur chirurgischen, ablativen, thermischen oder auch therapeutischen Laserbehandlung von Geweben des Auges, insbesondere der Hornhaut, der Sklera, des Trabekelwerks, der Netzhaut, der Linse oder des Glaskörpers.

Unabhängig von der Art der Behandlung, d. h. des zu behandelnden Gewebes, ist es bei dem Einsatz eines Lasers von besonderer Bedeutung entsprechende Vorschriften einzuhalten, um Verletzungen am Auge zu vermeiden. Neben der Laserleistung und der Gesamtstrahlenbelastung ist es dabei von besonderer Bedeutung, den Fokus des Behandlungslasers möglichst exakt auf das zu behandelnde Gewebe auszurichten, da Abweichungen unter Umständen zu Schäden an benachbarten Geweben des Auges führen könnten oder die angestrebte Behandlungswirkung am zu behandelnden Gewebe nicht erzielt würde. Beispielhaft wird dies an der Behandlung des Glaskörpers des Auges näher beschrieben.

Der Glaskörper besteht aus einer meist klaren, gelartigen Substanz im Augeninneren zwischen der Linse und der Netzhaut. Bei jungen Menschen ist der Glaskörper weitgehend durchsichtig und hat Kontakt zur Netzhaut. Im Laufe des Lebens verflüssigt sich der Glaskörper und löst sich zunehmend von der Netzhaut ab, was als hintere Glaskörperabhebung bezeichnet wird. Dies ist ein normaler Alterungsprozess, der sich üblicherweise nach dem 50. Lebensjahr abspielt. Glaskörperanteile können nach und nach im Inneren des Auges zusammenfallen und Gerüstsubstanzen und Verdichtungen des Glaskörpers werden für die Patienten zunehmend sichtbar. Da sie sich auch über das Gesichtsfeld bewegen können, werden sie auch als "Floater" bezeichnet. Oft liegen als Ursache von Glaskörpertrübungen nach der Ablösung des Glaskörpers auch membranartige Strukturen an der posterioren Seite des Glaskörpers vor, z.T. sogar Blutreste, falls es bei der Glaskörperablösung zu Netzhautverletzungen kam. In selteneren Fällen können Glaskörpertrübungen auch bei Stoffwechselproblemen als kristallartige Ausfällungen im Glaskörper vorliegen.

Auch wenn Glaskörpertrübungen meistens keine krankhafte Ursache haben, sind sie nicht so harmlos wie allgemein angenommen, weil sie die Lebensqualität und auch Arbeitsproduktivität der Betroffenen teilweise erheblich beeinträchtigen können. Besonders gegen einen hellen Hintergrund, z. B. beim Arbeiten am Computer, beim Lesen oder beim Blick gegen den blauen Himmel oder Schnee werden diese Trübungen wahrgenommen und stören das Sehvermögen. Besonders störend können Glaskörpertrübungen sein, die beim Lesen infolge der Lesebewegungen in den zentralen Sichtbereich hinein und hinaus geschleudert werden.

Weil sie oft die Form einer "fliegenden Mücke" haben, werden sie - aus dem Französischen - mit dem Fachausdruck "Mouches-Volantes" beschrieben. Die Trübungen können aber auch andere Formen haben, z. B. ast-, ring- oder sternförmig sein oder auch als Punktwolken vorliegen. Im Folgenden wird für die zu behandelnden Glaskörpertrübungen, unabhängig von deren Art bzw. Form der Begriff "Glaskörpertrübung" verwendet.

Glaskörpertrübungen verschwinden i.A. nicht ohne Behandlung, weil das Immunsystem sie nicht als abnormal erkennt und daher nicht abbaut. Von den Betroffenen lassen sie sich kaum ignorieren oder übersehen. Bestimmte Typen von Glaskörpertrübungen, wie diejenigen durch Blutreste nach Netzhautblutungen, werden z.T. vom Körper resorbiert, auch wenn dies oft Wochen oder Monate benötigt.

Bei einer sogenannten Vitrektomie wird nach Eröffnung des Auges mit Schneidinstrumenten der Glaskörper teilweise (Kernvitrektomie) oder vollständig zerkleinert, abgesaugt und entfernt. Ein derartiger Eingriff wird routinemäßig bei Netzhautablösungen oder Peeling epiretinaler Membranen durchgeführt, wird allerdings zur Beseitigung der umschriebenen Glaskörpertrübungen meist als unverhältnismäßige Therapie angesehen. Zudem ist die Vitrektomie invasiv, erfordert einen Klinikaufenthalt und birgt die mit einem chirurgischen Eingriff verbundenen Risiken, insbesondere häufig die Induzierung einer Katarakt, seltener einer Netzhautablösung und sehr selten, aber möglich einer Endophthalmitis.

Mit der sogenannten Laser-Vitreolyse bietet sich jetzt eine risikoarme Behandlungsalternative an. Die Laser-Vitreolyse ist eine schonende, risikoarme und schmerzfreie Laserbehandlung, mit der Glaskörpertrübungen vaporisiert oder zerstäubt werden können, ohne das Auge zu eröffnen.

Bei der Laser-Vitreolyse werden kurze Laserlichtpulse auf die Glaskörpertrübungen gerichtet, um dort auf Grund der hohen Laserintensität im Fokusbereich einen optischen Durchbruch bzw. eine Photodisruption zu erzielen. Die Glaskörpertrübungen und der sie umgebende Glaskörper nehmen die Laserenergie auf, es bildet sich ein schneidendes oder expandierendes Laserplasma, wodurch die Floater vaporisiert und/oder zerkleinert werden und sich dadurch auflösen können. Die Behandlung ist schmerzarm und ohne Infektionsrisiko. Mit der Laser-Vitreolyse steht ein sicheres Verfahren zur schonenden Behandlung von störenden Glaskörpertrübungen zur Verfügung, wenn sichergestellt werden kann, dass wichtige und empfindliche Augenstrukturen nicht durch den Laser geschädigt werden, wie z. B. der Kapselsack, die Linse oder Netzhautbereiche, insbesondere die Makula.

Insbesondere hierfür ist es wichtig den Behandlungslaserfokus möglichst genau auf die zu behandelnden Gaskörpertrübungen ausrichten zu können und eine fehlerhafte Ausrichtung des Behandlungslaserfokus auf benachbarte, möglicherweise empfindliche Augenstrukturen, wie Kapselsack, Nervenfaserkopf oder Makula zu vermeiden. Dabei ist wiederum die axiale Behandlungslaserfokusausrichtung besonders wichtig, da die zu behandelnden Glaskörpertrübungen und die zu schonenden Augenstrukturen i. A. in unterschiedlichen Tiefen im Auge liegen.

Allerdings ist der Erfolg der Behandlung von der Art der Glaskörpertrübungen abhängig. Besonders erfolgreich ist die Behandlung bei sogenannten Weiß-Ringen. Gewebestränge lassen sich durchtrennen und die Gewebsverdichtungen, die für die störenden Schatten verantwortlich sind, können beseitigt werden.

Bereits seit über 3 Jahrzehnten werden Glaskörpertrübungen mit YAG-Lasern (insbesondere als Nd:YAG bei 1064nm) behandelt (Brasse, K., Schmitz-Valckenberg, S., Jünemann, A. et al. Ophthalmologe (2019) 116: 73. https://doi.org/10.1007/s00347-018-0782-1). Aber selbst mit den bisherigen High-End Geräten kann nur der vorderste Bereich des Glaskörpers mit Präzision und Zielsicherheit behandelt werden. Im tieferen Glaskörperbereich sind diese Laser nicht präzise genug. Aber gerade dort liegen die meisten Glaskörpertrübungen, da sie ja oft das Resultat posteriorer Glaskörperablösungen sind. YAG-Laser werden in der Ophthalmologie häufig zur Iridotomie bei Glaukomerkrankungen und zur Nachstarbehandlung oder sogenannter Linsenpolitur eingesetzt, d.h. zur Entfernung von Trübungen oder gar einer Nachstarmembran auf Kunstlinsenimplantaten infolge von Zellüberwuchs. Frequenzverdoppelte YAG-Laser mit Laserstrahlung im Grünen (532nm) werden darüber hinaus auch zur Netzhautkoagulation eingesetzt, beispielsweise bei Blutungen oder Netzhautablösungen. Seltener kommen YAG-Laser auch zur Phakoemulsifikation bei einer Kataraktoperation zum Einsatz, also der Verflüssigung der eingetrübten und verhärteten natürlichen Linse. In diesem Fall aber eher als Er:YAG Laser mit 2940nm Wellenlänge und höherer Wasser- bzw. Gewebeabsorption, die dann auch oft aufwändig per endoskopischer Lasereinführung mit Spiegelführung in das Auge eingebracht werden müssen.

Nach dem bekannten Stand der Technik existieren bereits zahlreiche Lösungen zur Laserchirurgie von Gewebe des Auges, insbesondere im Glaskörper.

So beschreibt die DE 10 2011 103 181 A1 eine Vorrichtung und ein Verfahren zur Femtosekunden-Laserchirurgie von Gewebe, insbesondere im Glaskörper des Auges. Die Vorrichtung besteht aus einem Ultrakurzpulslaser mit Pulslängen im Bereich von ca. 10fs-1ps, insbesondere ca. 300fs, Pulsenergien im Bereich von ca. 5nJ-5uJ, insbesondere ca. 1-2µJ und Pulsrepetitionsraten von ca. 10kHz-10 MHz, insbesondere 500 kHz. Das Lasersystem ist mit einem Scanner-System gekoppelt, welches die räumliche Variation des Fokus in drei Dimensionen ermöglicht. Weiterhin ist eine Strahlführung durch ein optisches System vorgesehen, das die Scannerspiegel für die laterale Fokusverschiebung (x, y) in unmittelbare Nähe der Pupille des zu behandelnden Auges abbildet. Die Strahldivergenz kann dabei variiert werden, um eine Verschiebung der Fokusposition in axialer (z) Richtung zu realisieren. Die Vorrichtung besteht neben diesem therapeutischen Laser-Scanner-Optik-System weiterhin aus einem damit gekoppelten Navigationssystem.

Die US 2006/195076 A1 beschreibt System und Verfahren zur Herstellung von Einschnitten in Augengewebe in verschiedenen Tiefen. Das System und das Verfahren fokussieren Licht, möglicherweise in einem Muster, auf verschiedene Brennpunkte, die sich in verschiedenen Tiefen innerhalb des Augengewebes befinden. Mit einer segmentierten Linse können mehrere Brennpunkte gleichzeitig erstellt werden. Optimale Einschnitte können erzielt werden, indem das Licht nacheinander oder gleichzeitig in verschiedenen Tiefen fokussiert wird, eine erweiterte Plasmasäule und ein Strahl mit einer verlängerten Taille erzeugt werden. Die hier beschriebenen Techniken können unter anderem auch verwendet werden, um neue ophthalmologische Verfahren durchzuführen oder bestehende Verfahren zu verbessern, einschließlich Dissektion von Gewebe im posterioren Pol, wie beispielsweise Glaskörpertrübungen, Membranen und die Retina. Es wird in der Schrift erwähnt, dass bildgebende Verfahren, wie OCT oder Ultraschall, benutzt werden können, um die Lage und Dicke der Linse und des Kapselsacks zu bestimmen, um den Laser mit größerer Präzision fokussieren zu können. Weiter wird erwähnt, dass die Laserfokussierung durch direkte Beobachtung eines Ziellasers erfolgen kann (bekannt), aber auch dass die Laserfokussierung alternativ auch durch die direkte Beobachtung von OCT oder Ultraschall und andere medizinische Bildgebungsmodalitäten möglich wäre, was anzuzweifeln ist, weil ein Ziellaser eben nicht direkt im OCT oder Ultraschall beobachtet werden kann und es eben auch keine feste Lagebeziehung beispielsweise zwischen Laser und OCT gibt. Entsprechend wird eben auch nicht erklärt, wie die Bestimmung der Laserfokuslage per OCT oder Ultraschall oder andere medizinische Bildgebungsverfahren geschehen soll. Genau dieses Problem wird aber von dem in der vorliegenden Erfindung beschriebenen Verfahren für das dem OCT zugrundeliegende OCDR adressiert.

Auch die US 2014/257257 A1 beschreibt ein System und ein Verfahren zum Behandeln von Zielgewebe im Glaskörper eines Auges, umfassend eine Lasereinheit zum Erzeugen eines Laserstrahls und einen Detektor zum Erzeugen eines Bildes des Zielgewebes. Das System enthält auch einen Computer, der einen Brennfleckpfad zum Emulgieren des Zielgewebes definiert. Ein Komparator, der mit dem Computer verbunden ist, steuert dann die Lasereinheit, um den Brennpunkt des Laserstrahls zu bewegen. Diese Fokuspunktbewegung wird durchgeführt, um das Zielgewebe zu behandeln, während Abweichungen des Fokuspunkts vom definierten Fokuspunktpfad minimiert werden. Um die für die hierin beschriebenen Verfahren erforderliche Genauigkeit zu erreichen, wird die Behandlung mit einem computergesteuerten Laser durchgeführt, wobei die Kontrollreferenz vorzugsweise durch einen bildgebenden Detektor unter Verwendung einer Technik wie der optischen Kohärenztomographie (OCT) bereitgestellt wird. Wie die Lage des Fokus des Femtosekundenlasers zum OCT bestimmt wird, wird hier nicht beschrieben.

Die US 2015/342782 A1 betrifft ebenfalls ein System und ein Verfahren zur Verwendung eines computergesteuerten Lasersystems, um eine partielle Vitrektomie des Glaskörpers in einem Auge durchzuführen. Operativ wird zunächst ein optischer Kanal durch den Glaskörper definiert. Glasartige und suspendierte Ablagerungen (Glaskörpertrübungen) im optischen Kanal werden dann abgetragen und in einigen Fällen aus dem optischen Kanal entfernt (z. B. abgesaugt). In einigen Fällen kann eine klare Flüssigkeit in den optischen Kanal eingeführt werden, um das abgetragene Material zu ersetzen und dadurch eine ungehinderte Transparenz im optischen Kanal herzustellen. Allgemein betrifft die vorliegende Erfindung Systeme und Verfahren für ophthalmologische Laseroperationen. Insbesondere betrifft die vorliegende Erfindung Systeme und Verfahren zum Verwenden gepulster Laserstrahlen zum Entfernen sogenannter Glaskörpertrübungen. Auch bei dieser Lösung wird zur Erreichung der erforderlichen Genauigkeit eine Bildgebungseinheit verwendet, die in der Lage ist, ein dreidimensionales Bild anatomischer Merkmale im Auge zu erzeugen. Hierzu werden auf bekannten Techniken basierende Geräte vorgeschlagen, wie beispielsweise Scheimpflug-Geräte, konfokale Abbildungsgeräte, Ultraschallgeräte oder auch Bildgebungssysteme, die auf der optischen Kohärenztomographie (OCT) basieren. Wiederum wird in dieser Schrift aber nicht erklärt, wie die Lage des Laserfokus gegenüber dem OCT bestimmt wird.

Die US 2018/028354 A1 beschreibt ebenfalls ein Verfahren und ein System für einen ophthalmologischen Eingriff an einem Auge. Anhand eines Bildes von mindestens einem Teil des Auges werden unerwünschte Merkmale identifiziert. Dazu wird vorgeschlagen, die von einer auf die Pupille gerichteten Kamera aufgenommen Bilder oder auch die von einem OCT aufgenommen Bilder, die das Volumen des Auges zeigen, zu verwenden. Als unerwünschte Merkmale in der Glaskörperhöhle gelten glasige Trübungen, die die Sicht beeinträchtigen, wie beispielsweise Glaskörpertrübungen. Nach dem Identifizieren und Lokalisieren der Glaskörpertrübungen durch ein Bildverarbeitungssystem werden diese nach Bestätigung durch einen Arzt automatisiert mit Laserpulsen "beschossen". Die Laserenergie verdampft zumindest einen Teil einer glasartigen Opazität. Dieser Vorgang wird wiederholt, bis die Trübung des Glaskörpers beseitigt ist. Der gesamte Vorgang wird für jede Trübung des Glaskörpers wiederholt, bis die Flüssigkeit des Glaskörpers als ausreichend klar erachtet wird. Es wird in der Schrift "automatisiertes Zielen" mit dem Laser erwähnt, aber nicht, wie dieses erfolgt, insbesondere nicht, wie dies unter Verwendung von OCT-Bildern erfolgen soll.

Die EP 3 578 148 A1 schlägt vor, statt mittels einer aufwändigen Kalibration eine ausreichende Ausrichtung des Behandlungslaserfokus auf die zu bearbeitenden Gewebsstrukturen zu ermöglichen, in der Nähe der Bearbeitungszone zunächst mit dem Behandlungslaser Mikroinzisionen vorzunehmen und deren Lage zu erfassen und eine ggf. vorliegende Abweichung zwischen angestrebter und tatsächlicher Lage zu korrigieren. Dieser "Trial-and-error"-Ansatz belastet allerdings das Auge unnötig (unnötige Gewebsschädigung und Lichtbelastung mit Behandlungslaserlicht) und ist auch nicht immer durchführbar. Beispielsweise können keine stabilen Mikroinzisionen Behandlungszonennähe erzeugt werden, falls ein Auge teilweise oder vollständig flüssigkeitsgefüllt ist und trotzdem in der Flüssigkeit schwebenden Trübungen per Behandlungslaser zerstäubt werden sollen.

Ein von der Firma ELLEX beschriebenes Verfahren (Produkt-Broschüre der Firma Ellex Medical Pty Ltd.; "Tango Reflex - Laser Floater Treatment"; PB0025B; 2018; (http://www.ellex.com)) sieht die Nutzung eines gepulsten Nanosekundenlasers (YAG) vor, um Glaskörpertrübungen zu zerlegen oder durch Umwandlung in Gas vollständig zu beseitigen. Mit einem Pilot-Laserstrahl (d.h. Ziellaser im sichtbaren Spektralbereich, beispielsweise rot) wird das Zielgebiet (Glaskörpertrübung) anvisiert und danach mit ein oder mehreren Therapielaserpulsen "beschossen". Dabei werden sowohl der Pilot-Laserstrahl aus auch die Therapielaserpulse vom Anwender manuell ausgelöst. Eine derartige manuelle Laser-Behandlung besteht typischerweise aus zwei, jeweils 20-60 Minuten dauernden Einzelbehandlungen.

Die DE 10 2019 007 147 A1und DE 10 2019 007 148 A1 beschreiben Systeme zur Laser-Vitreolyse von Glaskörpertrübungen, die die sichere und präzise Zerstäubung von Glaskörpertrübungen ("Floatern") auf Basis einer Kombination eines Bearbeitungslasers mit einem OCT- oder einem OCDR-System ermöglichen. Unter einem OCDR- System (Optical Coherence Domain-Reflectometry) wird hierbei ein System zur interferometrischen Gewinnung von eindimensionalen Streuprofilen verstanden, während unter OCT (Optical Coherence Tomography) die 2- oder 3-dimensionale Bildgebung gemeint ist. In beiden Fällen sollen auch Varianten mit Aufnahmesequenzen (d.h. Film) mit eingeschlossen sein. Dabei werden Mindestabstände zu empfindlichen Augenstrukturen sichergestellt und die Aktivierung des Lasers bevorzugt nur dann erlaubt, wenn der Fokus des Bearbeitungslasers und die zu bearbeitende Glaskörpertrübung ausreichend genau zueinander positioniert sind.

Nachteilig wirkt sich bei diesen beiden Ansätzen aus, dass der Arzt die ihm zur Verfügung stehende, gewohnte 2-dimensionale Ansicht des Auges mit den 3-dimensionalen Aufnahmen des OCT- oder OCDR-Systems unter Einsatz seines räumlichen Vorstellungsvermögens ständig kombinieren muss, was bei der zeitkritischen bzw. schnellen Interaktion mit dem Patienten extrem schwierig ist. Dieser Nachteil wird durch die noch nicht veröffentlichten Patentanmeldung DE 10 2020 212 084.6 beschriebene Lösung behoben.

Die Verwendung von Laserenergie im Rahmen einer Laser-Vitreolyse ist nicht invasiv und vermeidet die Nachteile das Auge öffnender chirurgischer Eingriffe, ist jedoch auch mit Nachteilen bzw. Risiken verbunden.

So kann das Zielen des Lasers schwierig sein. Da der Arzt den Glaskörper entlang des Strahlengangs betrachtet, kann es schwierig sein, die Tiefe der Position der Netzhaut, die Tiefe der Trübung des Glaskörpers oder andere relevante Merkmale zu bestimmen. Infolgedessen besteht die Gefahr, dass die Trübung des Glaskörpers verfehlt und/oder das Auge verletzt wird.

Insbesondere erweist sich die Behandlung positionsveränderlicher und schwer erkennbarer, weitgehend transparenter Glaskörpertrübungen, die auch als Phasenobjekte noch störende Schatten auf der Netzhaut erzeugen können, als schwierig.

Die Anwendung von Laserenergie kann auch zu einer zusätzlichen Bewegung der Trübungen des Glaskörpers führen, was die Behandlung noch mehr erschwert. Somit kann es nötig sein, dass der Arzt den Laser nach jeder Anwendung von Laserenergie neu ausrichten muss. Dies kann viel Zeit in Anspruch nehmen. Daher ist eine Behandlung mit dem Laser aufwändig und sowohl für den Patienten als auch für den Arzt belastend.

Ein weiteres mögliches Problem sind unvollständige Glaskörperabhebungen, die zu lokalen Vitreotraktionen bis hin zu Netzhautablösungen führen können. Eine Laserbehandlung im Glaskörper kann durch die sich in ihrer Folge ausbreitenden Schockwellen zu Veränderungen der Kräfteverhältnisse im Glaskörper führen und dadurch beispielsweise Spannungen an der Netzhaut verursachen.

Letztendlich erweist sich auch die Behandlung von solchen Glaskörpertrübungen als besonders schwierig, die sich nahe an empfindlichen Strukturen des Auges befinden. Nicht nur die mechanische und thermische Belastung, sondern auch die Laserstrahlung selbst kann hier zu Schädigungen von Netzhaut, Augenlinse oder Macula führen und muss hinsichtlich ihrer Intensität und/oder Energie geeignet begrenzt werden.

Für diese obengenannten Probleme ist eine möglichst genaue Kenntnis der jeweilig momentanen Lage des Behandlungslaserfokus in Bezug auf Augenstrukturen immens wichtig. Klassisch wurde dazu für Nachstarbehandlungen oder Netzhautkoagulationen meist ein Ziellaser verwendet, der auf die jeweils zu behandelnde Gewebsstruktur ausgerichtet wurde, indem der Bediener die Rückstreuung der Ziellaserstrahlung beobachtet. Das ist allerdings nahezu unmöglich, wenn die zu behandelnden Strukturen beispielsweise sehr schwach streuende Objekte sind, die eigentlich nur noch mittels hochempfindlicher Systeme, wie OCT oder OCDR erfasst werden können. Übliche OCT und OCDR Systeme können beispielsweise Empfindlichkeit von über 85, 90, 100 oder 110dB aufweisen, wobei ab ca. 90dB eine Detektion der normalen Glaskörperrückstreuung möglich wird, also auch die Erfassung von Glaskörpertrübungen.

Werden aber keine Ziellaser verwendet, sondern OCT-gestützte Systeme, besteht das komplexe Problem einer ausreichend genauen Zuordnung der Behandlungslaserfokuslage zu einer Position im OCT oder OCDR-Scan. Dies liegt darin begründet, dass die axiale Behandlungslaserfokuslage sich schon durch Variationen von Brechkräften (beispielsweise Oberflächenkrümmungen) im Strahlweg deutlich verändern kann, während diese im OCT bzw. OCDR nahezu keinen Einfluss auf die Positionen von Signalen streuender Objekte im OCT oder OCDR haben. YAG-Fokusdurchmesser und auch axiale Rayleigh-Längen im Augeninneren liegen ca. in der Größenordnung von 20µm, so dass schon kleine Abweichungen problematisch sind, wenn feine Objekte mit ähnlichen räumlichen Dimensionen bearbeitet werden sollen. Insofern können schon kleine Änderungen, beispielsweise infolge thermischer Effekte im Laser während längeren Betriebs, einen relevanten Einfluss haben und eine Rekalibrierung der Fokuslage gegenüber dem OCDR erfordern.

Ein Teil der nach dem Stand der Technik bekannten Lösungen unter Verwendung von OCT-Systemen gehen von bekannten Brennweiten, Brechkräften, Abstandsparametern und Probenlagen aus, so dass eine Berechnung der Fokuslage in Bezug auf das OCT oder OCDR theoretisch möglich wäre. Dazu müssten diese Parameter allerdings immer ausreichend genau bekannt sein oder mit hoher Genauigkeit bestimmt werden, was jedoch insbesondere in Hinblick auf die Bestimmung aller relevanten Brechkräfte (wie der Hornhaut- und Linsenflächen) am Auge immens aufwändig wäre. Auch können prä-operativ gewonnene Daten unzureichend sein, da sich beispielsweise im Zuge der Verwendung, beispielsweise handgehaltener Kontaktgläser, anpressdruckabhängige Änderungen von Hornhautbrechkräften ergeben können. Auch variieren die menschlichen Augenanatomien beträchtlich. Beispielsweise treten schon Augenlängen im Bereich von 14 ... 40mm auf, sowie sehr unterschiedlich tiefe Vorderkammern (1,5 bis 4 mm) oder Linsendicken (natürliche Linsen: ~3 ... 4mm, IOLs z.T. dünner oder z.T. auch dicker, falls beispielsweise mehrteilige IOLs benutzt werden).

Neben den unterschiedlichen Fokustiefen und den variierenden Brechkräften der individuellen Augen können auch Toleranzen der Optik des Lasersystems zu zusätzlichen Abweichungen der Fokuslage des Behandlungslasers führen.

Auch das in DE 10 2019 007 147 A1 vorgeschlagene Konzept, die Fokuslage von OCDR und Behandlungslaser gemeinsam zu variieren (d.h. durchzustimmen) und aus den resultierenden OCDR-Signalvariationen die Kalibrierung zwischen OCDR und Fokus des Behandlungslasers abzuleiten, benötigt eine ausreichend fein veränderbare Fokuslage bei gleichzeitiger, synchronisierter OCDR-Erfassung und wäre insbesondere auch etwas aufwändiger, falls unterschiedliche axiale Fokuslagen von OCDR und Behandlungslaser genutzt werden sollen (also ein Versatz zwischen den Foki vorliegen soll). Zusätzlich wird dieser Ansatz dadurch erschwert, dass die OCDR- oder OCT-Aufnahmen durch ihre kohärenten Eigenschaften "verspeckelt" sind und sich so der Punkt des größten Signales um den Fokus manchmal nur mit etwas Zusatzaufwand (beispielsweise durch Speckle-Unterdrückung, wie in US 8,085,408 B2 oder DE10 2008 051 272 A1) bestimmt werden kann.

Deshalb ist es in OCDR oder OCT-gestützten Systemen von besonderer Bedeutung, eine möglichst exakte Einschätzung der Fokuslage des Behandlungslasers gegenüber dem zu behandelnden Gewebes und den zu schonenden Augenstrukturen zu ermöglichen. Die nach den Lösungen des Standes der Technik vorgenommenen Kalibrierungen, insbesondere mittels eines Testauges oder angenommener bzw. vorbestimmter Parameter, haben sich dabei bislang als unzureichend oder sehr aufwändig erwiesen.

Der vorliegenden Erfindung liegt deshalb die Aufgabe zugrunde, eine Lösung für ein ophthalmologisches System zur intraokularen Laserbehandlung zu entwickeln, die die Nachteile der bekannten technischen Lösungen behebt, indem sie die Individualität eines zu behandelnden Auges und die Toleranzen des optischen Systems durch Rekalibrierung des Fokus des Behandlungslasers berücksichtigt. Damit soll es insbesondere auch möglich sein, die Fokuslage des Behandlungslasers gegenüber OCT bzw. OCDR-Scans für jede neue Behandlungssituation, und zwar insbesondere nach Wechsel des Patienten und/oder von Kontaktgläsern oder auch einer Veränderung der Brennweite des Behandlungslasers, des Akkommodationszustandes des Patienten und/oder des Probenabstandes, zu kalibrieren.

Zudem soll die Lösung leicht zu implementieren und wirtschaftlich kosten-günstig sein und eine einfachere, schnellere und vor allem sicherere Laserbehandlung am Auge ermöglichen.

Diese Aufgabe wird durch die Anordnung nach Anspruch 1 gelöst. Vorteilhafte Ausgestaltungen sind Gegenstand der Unteransprüche.

Genauer wird die Aufgabe gelöst durch eine Anordnung zur Rekalibrierung des Fokus eines ophthalmologischen Systems zur intraokularen Laserbehandlung bestehend aus einer Behandlungslasereinheit, einer Bildgebungseinheit und einem optischen System zur Fokussierung und Strahlüberlagerung sowie einem OCDR-System und einer Steuereinheit. Das Laserbehandlungssystem ist so ausgebildet, dass der Abstand A zum Auge veränderbar und ein Ziellaserstrahl auf mindestens eine Zielstruktur ZS1 im zu behandelnden Auge fokussierbar ist. Ferner ist die Steuereinheit ausgebildet, einen Abstand A1 aus der Position einer gewählten Referenzstruktur PRS, sowie die Position der Zielstruktur PZS1 im OCDR-Signalprofil jeweils bezogen auf eine Referenzebene RE zu bestimmen, und ist weiter ausgebildet, für beliebig wählbare Werte einer Abstandsveränderung ΔA des Laserbehandlungssystems zum Auge die jeweils anzunehmende Fokusposition PF des Laserstrahls des Laserbehandlungssystems im OCDR-Signalprofil näherungsweise unter Verwendung der Parameter A1 und PZS1 abzuschätzen.

Nachfolgend wird zum besseren Verständnis ein Verfahren beschrieben, welches mit der erfindungsgemäßen Anordnung ausführbar ist, wobei das Verfahren kein Teil der Erfindung ist. Genauer handelt es sich um dabei um ein Verfahren zur Rekalibrierung des Fokus eines ophthalmologischen Systems zur intraokularen Laserbehandlung, welches über eine Behandlungslasereinheit, eine Bildgebungseinheit und ein optisches System zur Fokussierung und Strahlüberlagerung auch über ein OCDR-System und eine Steuereinheit verfügt, dadurch gelöst, dass ein Ziellaserstrahl des Laserbehandlungssystems auf mindestens eine Zielstruktur ZS₁ im zu behandelnden Auge fokussiert wird, indem der Abstand A des Laserbehandlungssystems zum Auge verändert wird, bis die Fokussierung des Ziellaserstrahls des Laserbehandlungssystems auf die Zielstruktur ZS₁ detektiert wird, dass ein Abstand A₁ aus der Position einer gewählten Referenzstruktur PRS, sowie die Position der Zielstruktur PZS₁ im OCDR-Signalprofil jeweils bezogen auf eine Referenzebene RE bestimmt wird und dass für beliebig wählbare Werte einer Abstandsveränderung ΔA des Laserbehandlungssystems zum Auge die jeweils anzunehmende Fokusposition PF des Laserstrahls des Laserbehandlungssystems im OCDR-Signalprofil näherungsweise unter Verwendung der Parameter A₁ und PZS₁ abgeschätzt wird.

Einer ersten vorteilhaften Ausgestaltung entsprechend wird der Laserstrahl des Laserbehandlungssystems (bzw. ein Substitut des Laserstrahls, wie eines Ziellaserstrahls oder eines abgeschwächten Behandlungslaserstrahls) auf mindestens eine erste Zielstruktur ZS₁ im zu behandelnden Auge fokussiert, indem der Abstand A des Laserbehandlungssystems zum Auge zwischen einer Referenzebene RE bei einem Abstand ΔE vor einer Bezugsebene BE des Lasersystems (beispielsweise dem Frontlinsenscheitel) und eine Referenzstruktur RS am Auge (beispielsweise dem Hornhautscheitel) verändert wird, bis bei einer Position A₁ die Fokussierung des Laserstrahls auf die Zielstruktur ZS₁ erkennbar ist, beispielsweise an der stärksten Rückstreuung des Laserstrahls des Laserbehandlungssystems oder des Zielstrahls von der Zielstruktur, dass die (korrespondierende) Position PZS₁ dieser Zielstruktur im OCDR-Signal bestimmt wird, dass bei Fokussierung auf weitere Zielstrukturen ZSₙ (n=2...N) die jeweils dazu nötige Abstandsveränderung ΔAₙ gegenüber der Position A₁ der Fokussierung auf die erste Zielstruktur ZS₁ bestimmt wird, dass bei jeder Fokussierung auf eine der Zielstrukturen ZSₙ jeweils deren Position PZSₙ im OCDR bestimmt wird, und dass für andere, beliebig wählbare Werte einer Abstandsveränderung ΔA des Laserbehandlungssystems zum Auge die jeweils anzunehmende Fokusposition PF des Laserstrahls des Laserbehandlungssystems näherungsweise aus der Funktion $PF \left(ΔA\right) = {f}_{{A}_{1} , {ΔA}_{2} … {ΔA}_{N} , {PZS}_{1} … {PZS}_{N}} \left(ΔA\right)$ (1) bestimmt wird. "Beliebig wählbare Werte" bedeutet hierbei praktisch realisierbare Abstandszustände zwischen Auge und Lasersystem. Als Zielstrukturen eignen sich in phaken Augen beispielsweise die Vorder- und Rückseite der Linse, in aphaken Augen die IOL-Vorder- oder Rückseite oder zu einem gewissen Grad auch eine IOL-Haptik (bevorzugt flächige Kunststoffhaptik).

Generell sind auch Vorder- und Rückseite des Kontaktglases oder der Hornhaut, sowie die Netzhautoberfläche geeignete Zielstrukturen. Wenn Glaskörpertrübungen den Ziellaserstrahl stark genug zurückstreuen, sind diese ebenfalls als Zielstrukturen geeignet. Generell ist es vorteilhaft, wenn Zielstrukturen verwendet werden, die möglichst "nahe" am Bearbeitungsgebiet liegen (axial, aber auch lateral), um eine möglichst hohe Genauigkeit der Fokuspositionsannahme PF(ΔA) zu erreichen, beispielsweise in einer Nähe von weniger als 2mm, bevorzugt aber weniger als 1mm und noch bevorzugt weniger als 100µm.

Erfindungsgemäß beträgt die Abweichung der näherungsweisen Bestimmung der anzunehmenden Fokusposition PF des Laserstrahls von der tatsächlichen Lage dabei weniger als 2 Rayleigh-Längen des LaserFokus, insbesondere weniger als 1 Rayleigh-Länge oder besonders bevorzugt, weniger als 0,5 der Rayleigh-Länge. Die Rayleigh-Länge ergibt sich aus z_{R}=n*π*w₀²/M², wobei n der Brechzahl des Mediums, w₀ dem Radius des Laserstrahls im Fokus und 1/M² der Strahlqualität (idealerweise M=1) entsprechen. Die doppelte Rayleigh-Länge entspricht dabei der bei Materialbearbeitungslasern üblichen Definition der Schärfentiefe (A. Barz, H. Müller, J. Bliedtner, "Lasermaterialbearbeitung", Hanser Fachbuchverlag; https://www.hanser-fachbuch.de/buch/Lasermaterialbearbeitung/9783446421684) und liegt bei Laseranwendungen am Auge beispielsweise zwischen 9µm (eher oberflächennahe Anwendung mit großer numerischer Apertur) und 1mm. Prinzipiell ist es auch möglich, das Verfahren für einen, aus dem Stand der Technik bekannten, mehrteiligen Behandlungslaserfokus zu nutzen. In diesem Fall sind dann allerdings alle Fokusteile einzeln zu kalibrieren, per umschaltbaren oder mehrteiligen Ziellaser, oder aber zumindest ein ausgewählter Behandlungslaserfokusteil, so dass für die anderen Fokusteile dann eine Lageabschätzung basierend auf der Rekalibrierung des ausgewählten Fokusteils realisiert werden kann.

Bevorzugte Weiterbildungen und Ausgestaltungen sind Gegenstand der abhängigen Ansprüche.

Einer zweiten Ausgestaltung entsprechend wird statt dem Laserstrahl des Laserbehandlungssystems der Kreuzungspunkt von Ziellasern auf mindestens eine Zielstruktur ZS im zu behandelnden Auge fokussiert. Insbesondere wird als Ziellaser ein cw-Laserstrahl mit gleicher oder ähnlicher Fokuslage wie der Laserstrahl des Laserbehandlungssystems verwendet. Die Fokussierung des Ziellasers auf die einzelnen Zielstrukturen ZSₙ kann dabei durch Erfassen der maximalen Rückstreuung der Ziellaserstrahlung von der jeweilig "anfokussierten" Zielstruktur per Bedienerauge oder per Kamera mit Bildverarbeitung erfasst werden, letzteres insbesondere, falls nichtsichtbare Ziellaserstrahlung genutzt werden soll, beispielsweise im NIR bei 800 oder 1060nm.

Bei dem vorgeschlagenen Verfahren dienen als Zielstrukturen ZSₙ die Linsenrückseite (Linse oder IOL), die Kapselsackrückseite, die Retinaoberfläche oder andere Strukturen des Auges.

Einer dritten Ausgestaltung entsprechend wird mindestens eine zumindest kurzfristig stabile Zielstruktur ZS durch den Laserstrahl des Laserbehandlungssystems oder auch den Ziellaser erzeugt, beispielsweise durch einen Laserschuss des Laserbehandlungssystems oder auch durch einen modulierten Ziellaser, indem eine zumindest für eine Kalibrierung ausreichend detektierbare Veränderung im Auge bewirkt wird, die im OCDR eine charakteristische, messbare Signaländerung bewirkt.

Die Modulation des Ziellasers erfolgt dabei beispielsweise akusto-optisch, elektro-optisch, interferometrisch bzw. wellenlängen- oder polarisationsmoduliert. Es ist aber auch möglich den Ziellaser beispielsweise über Strommodulation oder veränderbare Abschwächer, wie einem Filterrad, einem Chopper oder ähnlichem zu modulieren. Eine damit aufgeprägte charakteristische Lasermodulation kann dann mittels einer auf diese Modulation abgestimmte Filterung des OCDR-Signals in den Speckle-Variationen wieder detektiert werden, um die Position der des Laserfokus im OCDR-Signal sehr genau zu bestimmen.

Alternativ können beispielsweise auch abgeschwächte Schüsse des Laserbehandlungssystems durch Plasmaexpansion im OCDR zumindest kurzfristig erfassbare Gasblasen erzeugen (bevor diese aus dem OCDR-Strahl heraus aufsteigen) oder auch ein modulierter Ziellaserstrahl im Fokussierungsgebiet durch Lichtabsorption und lokale Erwärmung eine lokale modulierte Phasenmodulation bzw. Specklevariationen im OCDR oder OCT Signal bewirken. Für letzteres ist es günstig, wenn der Ziellaser den OCDR- bzw. OCT-Strahl im Fokusbereich unter einem Winkel kreuzt, um dort ein maximales transientes Signal der Zielstruktur zu erzeugen. Die Modulationsfrequenz darf dabei allerdings nicht zu hoch sein, damit die Wärmeableitung noch Temperaturmodulationen ermöglicht. Mögliche Modulationsfrequenzen liegen im Bereich von 0,1 ... 100 Hz, günstige im Bereich von 5 ... 50 Hz. Günstig Ziellaserwellenlängen liegen im NIR, wie 1,2 ... 1,7 µm, insbesondere 1,3 µm und 1,5 µm, bei denen Wasser gut absorbiert und die Lichtbelastung der Patientenretina gering ist.

Gegenüber dem Stand der Technik sind mit diesem Ansatz beliebig oft wiederholbare Fokuskalibrierungen auch in instabilen Medien ohne natürliche Zielstrukturen möglich, wie im Kammerwasser der Vorderkammer oder auch in einem teilverflüssigten Glaskörper oder in einer diesen ersetzenden Kochsalzwasserlösung nach einer Vitrektomie.

In diesem Zusammenhang ist es vorteilhaft, dass die durch einen Laserschuss des Laserstrahls des Laserbehandlungssystems erzeugte Zielstruktur ZS neben der Bestimmung der Fokuslagen auch zur Titration der Laserleistung genutzt werden kann.

Einer vierten Ausgestaltung entsprechend dienen als Referenzstruktur RS zur Bestimmung der jeweiligen Abstände A zwischen Auge und Lasersystem die Vorder- oder Rückseite eines vorhandenen Kontaktglases KG, eine im Kontaktglas befindliche technische Struktur oder aber natürlicher oder künstlicher Augenstrukturen, wie die Vorder- oder Rückseite von Hornhaut, Linse oder IOL, Kapselsack oder die Retinaoberfläche.

Die im Kontaktglas KG realisierte Referenzstruktur RS erzeugt dabei ein im OCDR charakteristisches Signal, wobei die realisierte Referenzstruktur RS vorzugsweise veränderbar, insbesondere schaltbar oder modulierbar ist.

Einer besonders vorteilhaften, fünften Ausgestaltung entsprechend werden der Laserstrahl des Laserbehandlungssystems bzw. der Kreuzungspunkt von Ziellasern auf eine Anzahl N von Zielstrukturen ZSₙ im zu behandelnden Auge fokussiert, um für beliebig wählbare Werte einer Abstandsveränderung ΔA die jeweils anzunehmende Fokusposition PF des Laserstrahls des Laserbehandlungssystems aus der Funktion (1) möglichst genau abzuschätzen. Die als Index der Funktion angegebenen Parameter sind mit A₁, ΔAₙ=_{2...N} die Augenabstände bzw. deren Änderungen gegenüber A₁, bei denen jeweils auf Zielstrukturen ZSₙ fokussiert wird und mit PZSₙ die dafür jeweils ermittelten Zielstruktursignalpositionen im OCDR. ΔA₁ wird hier als Parameter nicht mehr mit aufgeführt, da A₁ der Bezugsabstand für alle Abstandsänderungen ΔA ist und deshalb eigentlich ΔA₁=0 gilt. Würde ein anderer Bezugsabstand als A₁ gewählt, könnte natürlich als Parameter statt A₁ dann auch dessen Abstand ΔA₁ zum Bezugsabstand statt A₁ als Parameter der Funktion verwendet werden. Für die Funktion ${f}_{{A}_{1} , {ΔA}_{2} … {ΔA}_{N} , {PZS}_{1} … {PZS}_{N}} \left(ΔA\right)$ wird zur Bestimmung der Fokuspositionen PF(ΔA) bevorzugt ein Polynom ersten bis N-ten Grades (oder eine andere nichtlineare Funktion mit N Freiheitsgraden, beispielsweise eine Fourier-Reihe) genutzt, welches so gewählt ist, dass es mit möglichst kleinen Abweichungen durch die Punkte ΔA_{n=1...N}, PZS_{n=1...N} verläuft. Dies kann beispielsweise durch ein Anfitten des Polynoms oder der nichtlinearen Funktion an die Punkte erfolgen, beispielsweise durch Anwendung der Methode der kleinsten Quadrate.

Die Funktion (1) zur Bestimmung der Fokuspositionen **FP** kann aber auch ein Polynom oder eine nichtlineare Funktion höheren Grades als N sein und indem zur Bestimmung des Polynoms oder der nichtlinearen Funktion zusätzliche Parameter des Kontaktglases oder des Auges herangezogen werden.

Einer sechsten Ausgestaltung entsprechend wird das Bildgebungssystem zusammen mit dem Laserstrahl des Laserbehandlungssystems bzw. dem Ziellaser schrittweise auf die Zielstrukturen ZSₙ fokussiert, wofür ein Autofokussystems verwendet wird. Insbesondere weisen das Bildgebungssystem und der Laserstrahl des Laserbehandlungssystems dabei eine numerische Apertur auf (NA), die sich um weniger als einen Faktor 2 unterscheiden. Besonders vorteilhaft ist hierbei, wenn das Bildgebungssystem und der Laserstrahl des Laserbehandlungssystems Wellenlängen aufweisen, die höchstens 10% voneinander abweichen.

Einer siebenten Ausgestaltung entsprechend dient das Verfahren der Rekalibrierung des Fokus eines Behandlungssystems zur Laser-Vitreolyse, welches neben einer Fokussiereinheit auch über ein OCDR-System und eine Steuereinheit verfügt.

Der Laserstrahl des Laserbehandlungssystems wird dabei auf zwei oder mehr Zielstrukturen ZSₙ (n=2...N) im zu behandelnden Auge fokussiert, indem nach der Fokussierung auf die erste Zielstruktur ZS₁ der Abstand A des Behandlungssystems zur Laser-Vitreolyse ausgehend von einer Referenzebene RE in Bezug auf das Auge um Änderungen ΔA verändert wird, bis die Fokussierung des Laserstrahls auf die jeweilige Zielstruktur ZSₙ anhand der maximierten Rückstreuung des Behandlungslasers oder Zielstrahllaser erkennbar ist und dass für diese Fokussierungssituationen die vorliegende Abstandsveränderung ΔAₙ bestimmt wird, dass für diese Fokussierungssituation die Position PZSₙ der Zielstruktur ZSₙ, auf die bei ΔAₙ gerade fokussiert wird, aus dem OCDR-Signal bestimmt wird und dass für andere beliebig wählbare Werte einer Abstandsveränderung ΔA die jeweils anzunehmende Fokusposition PF des Laserstrahls des Behandlungssystems zur Laser-Vitreolyse näherungsweise aus der Funktion (1) bestimmt wird. Als Zielstrukturen ZSₙ werden bevorzugt jeweils eine Struktur im vorderen und im hinteren Augenbereich, vorzugsweise die Linsenrückseite und die Retinaoberfläche ausgewählt.

Die näherungsweise Bestimmung von Positionen des Behandlungslaserfokus $PF \left(ΔA\right) = {f}_{{A}_{1} , {ΔA}_{2} … {ΔA}_{N} , {PZS}_{1} … {PZS}_{N}} \left(ΔA\right)$ für beliebige Abstände ΔA des Lasersystems zum Auge wird dann insbesondere zur Feststellung der Behandlungslaserfokusposition (für den Zeitpunkt der Auslösung des Laserschusses) relativ zu den Sperrzonen eines Behandlungssystems für Glaskörpertrübungen und einer daraus resultierenden Aktivierung oder Deaktivierung des Therapielasers genutzt. Es ist hierbei auch möglich Augenbewegungen, insbesondere axiale, beispielsweise per OCDR zu erfassen, und diese dann bei einer latenzzeitbehafteten Behandlungslaserfokuspositionsabschätzung korrigierend zu berücksichtigen.

Besonders vorteilhaft ist es, wenn mindestens eine Zielstruktur ZS in der Nähe einer zu bearbeitenden Augenstruktur gewählt wird. Hierbei besteht auch wieder die Möglichkeit, eine permanente oder transiente Zielstruktur per (abgeschwächtem) Behandlungslaser oder per Zielstrahllaser zu generieren.

Bei Behandlungssystemen zur Laser-Vitreolyse ist zu gewährleisten, dass in Abhängigkeit von der Bearbeitungstiefe ein jeweils ausreichend großer Pupillendurchmesser vorliegt, um eine ausreichend kurze Schärfentiefe des Behandlungslasers sicherzustellen. Um dies zu sichern, wird vorzugsweise ein Bildgebungssystem für den Pupillenbereich genutzt, mit dessen Hilfe der momentan vorliegende Pupillendurchmesser bestimmt wird. Alternativ kann auch die Rückstreuung eines dem Behandlungslaserstrahl entsprechenden Ziellasers am Rand einer unzureichend geweiteten Pupille als Kriterium genutzt werden. Für eine Laser-Vitreolyse Behandlung im anterioren Bereich sind Pupillendurchmesser >4mm, im zentralen Bereich >5mm und im posterioren Bereich >6mm günstig. Das Behandlungssystem verhindert die Auslösung des Behandlungslasers und warnt, wenn diese Bedingungen nicht erfüllt sind. Ggf. muss dann das Umgebungslicht im Behandlungsraum reduziert werden oder medikamentös eine Pupillendilatation bewirkt werden.

Das Verfahren zur Rekalibrierung des Fokus eines ophthalmologischen Systems zur intraokularen Laserbehandlung ist insbesondere zur Realisierung eines Behandlungssystems zur Laser-Vitreolyse vorgesehen.

Mögliche andere intraokuläre Anwendungsmöglichkeiten, die von dem Verfahren zur Rekalibrierung des Fokus eines Laserbehandlungssystems profitieren würden, sind beispielsweise folgende, woraus sich jedoch kein Anspruch auf Vollständigkeit ergibt:
- Netzhautkoagulationen
- SRT (Selektive Retina Therapie)
- Glaskörperschnitte, z.B. zur Behandlung von Vitreotraktionen
- Lasertrabekuloplastien (wie SLT) oder -tomien (wie ALT)
- Nachstarbehandlungen
- Femtosekundenkatarakt-OPs (Rhexis und Linsenschnitte) und
- Schnitte oder Ablationen an der Hornhaut.

Die Erfindung wird nachfolgend anhand von Ausführungsbeispielen näher beschrieben. Dazu zeigen
- Figur 1:: das auf eine erste Zielstruktur ZS₁ im zu behandelnden Auge fokussierte Behandlungssystem zur Laser-Vitreolyse,
- Figur 2:: das auf eine zweite Zielstruktur ZS₂ im zu behandelnden Auge fokussierte Behandlungssystem zur Laser-Vitreolyse,
- Figur 3:: ein Behandlungssystem zur Laser-Vitreolyse, welches ein Kontaktglas mit einer Referenzstruktur RS nutzt und
- Figur 4:: die Funktion PF(ΔA) zur Bestimmung beliebiger Fokuspositionen PF des Laserstrahls des Laserbehandlungssystems.

Das Verfahren zur Rekalibrierung des Fokus eines ophthalmologischen Systems zur intraokularen Laserbehandlung verfügt über eine Behandlungslasereinheit, eine Bildgebungseinheit und ein optisches System zur Fokussierung und Strahlüberlagerung auch über ein OCDR-System und eine Steuereinheit.

Erfindungsgemäß wird der Laserstrahl des Laserbehandlungssystems auf mindestens eine erste Zielstruktur ZS₁ im zu behandelnden Auge fokussiert, indem der Abstand A₁ des Laserbehandlungssystems zum Auge verändert wird, bis die Fokussierung des Laserstrahls auf die Zielstruktur ZS₁ erreicht ist. Der Abstand A₁ wird aus der Position einer gewählten Referenzstruktur PRS und der Position der Zielstruktur PZS₁ im OCDR-Signalprofil bezogen auf eine Referenzebene RE bestimmt. Für beliebig wählbare Werte einer Abstandsveränderung ΔA des Laserbehandlungssystems zum Auge lässt sich die jeweils anzunehmende Fokusposition PF des Laserstrahls des Laserbehandlungssystems im OCDR-Signalprofil näherungsweise unter Verwendung der Parameter A₁ und PZS₁ abschätzen.

Die Fokussierung des Ziellasers auf die Zielstruktur ZS₁ kann dabei durch Erfassen der maximalen Rückstreuung der Ziellaserstrahlung von der "anfokussierten" Zielstruktur ZS₁ per Bedienerauge oder per Kamera mit Bildverarbeitung erfasst werden. Dann wird die Position des OCDR-Signals PZS₁ der Zielstruktur ZS₁ bestimmt.

Bevorzugt werden die ermittelten Werte A₁ und PZS₁ verwendet, eine Funktion $PF \left(ΔA\right) = {f}_{{A}_{1} , {PZS}_{1}} \left(ΔA\right)$ zu bestimmen, die für beliebig wählbare Werte einer Abstandsveränderung ΔA des Laserbehandlungssystems zum Auge die jeweils anzunehmende Fokusposition PF des Laserstrahls des Laserbehandlungssystems im OCDR-Signalprofil näherungsweise berechnet.

Sollen zur Erhöhung der Kalibrierungsgenauigkeit über große Tiefenbereiche weiteren Zielstrukturen Z₂ bis Z_{N} in gleicher Weise "anfokussiert" werden, so werden dafür jeweils die Abstandsveränderungen ΔA₂ bis ΔA_{N} eingestellt, bei denen auf diese Zielstrukturen mit dem Laserstrahl des Laserbehandlungssystems oder mit dem Ziellaser jeweils bestmöglich fokussiert wird, sowie dabei vorliegenden Positionen der Zielstruktursignale PZS₂ bis PZS_{N}. Aus den so bestimmten Werten A₁ und PZS₁ und ggf. ΔA₂ bis ΔA_{N} und PZS₂ bis PZS_{N} kann dann die Funktion PF(ΔA)= *f*_{*A*₁*,*ΔA₂ *...* ΔA*N,PZS*₁*...* PZS*_{N}*}(Δ*A*) bestimmt werden, die für andere beliebig wählbare Werte einer Abstandsveränderung ΔA dann die jeweils anzunehmende Fokusposition PF des Laserstrahls des Laserbehandlungssystems näherungsweise bestimmt.

Wie schon oben erwähnt, sind die als Index angegebenen Parameter A₁, ΔA_{n=2... N} die Augenabstände bzw. deren Änderung gegenüber A₁, bei denen jeweils auf Zielstrukturen ZSₙ fokussiert wird, und PZSₙ die dafür jeweils ermittelten Zielstruktursignalpositionen im OCDR. Für die Funktion (1) wird zur Bestimmung der Fokuspositionen $PF \left(ΔA\right) = {f}_{{A}_{1} , {ΔA}_{2} … {ΔA}_{N} , {PZS}_{1} … {PZS}_{{N}_{□}}} \left(ΔA\right)$ bevorzugt ein Polynom ersten bis N-ten Grades oder eine andere nichtlineare Funktion mit N Freiheitsgraden genutzt, welches so gewählt ist, dass es mit möglichst kleinen Abweichungen durch die Punkte ΔA_{n=1...N}, PZS_{n=1... N} verläuft. Dies kann beispielsweise durch ein Anfitten des Polynoms oder der nichtlinearen Funktion an die Punkte erfolgen, beispielsweise durch Anwendung der Methode der kleinsten Quadrate.

Bevorzugter Weise erfolgt die Fokussierung des Laserstrahls des Laserbehandlungssystems auf mindestens eine Zielstruktur ZS im zu behandelnden Auge mit dem Behandlungslaser mit reduzierter Pulsenergie, um eine Photodisruption zu vermeiden. Es ist auch möglich, dass als Ziellaserstrahl ein zusätzlicher Laserstrahl genutzt wird, dessen Parameter keine permanente Gewebsveränderung im Auge zulässt.

Erfindungsgemäß beträgt die Abweichung der näherungsweisen Bestimmung der anzunehmenden Fokusposition PF des Laserstrahls von der tatsächlichen Lage weniger als 2 Rayleigh-Längen des Laserfokus, insbesondere weniger als 1 Rayleigh-Länge oder besonders bevorzugt, weniger als 0,5 der Rayleigh-Länge.

Falls das Laserbehandlungssystem über mehrere Ziellaser verfügt, kann statt des Laserstrahls des Laserbehandlungssystems der Kreuzungspunkt der Ziellaser auf mindestens eine Zielstruktur ZS im zu behandelnden Auge fokussiert werden. Dieser kann kontinuierlich oder quasikontinuierlich oder ggf. gepulst, die Position des Fokus des Laserstrahls des Laserbehandlungssystems anzeigen. Dazu kann ein Zielstrahllaser im sichtbaren Spektralbereich benutzt werden, oder bei Nutzung eines Kamerasystems, beispielsweise auch im **NIR. Um** den Ort des Fokus des Laserstrahls des Laserbehandlungssystems anzuzeigen, kann der Ziellaser beispielsweise kollinear mit dem Laserstrahl des Laserbehandlungssystems überlagert sein und eine gleiche Fokuslage besitzen. Wird dann damit auf eine Zielstruktur fokussiert, ist dies an einem minimal großen und maximal intensiv rückstreuenden Ziellaser-Fleck auf der Zielstruktur erkennbar (detektierbar per Auge im VIS oder per Kamera im NIR). Alternativ können auch mehrere Ziellaserstrahlen genutzt werden, die sich am Ort des Fokus des Laserstrahls des Laserbehandlungssystems kreuzen. Weiter alternativ können ein oder mehrere sich bewegende, beispielsweise rotierende Ziellaserstrahlen genutzt werden, die jeweils durch den Ort des Fokus des Laserstrahls des Laserbehandlungssystems verlaufen.

Die Detektion einer Fokussierung auf eine Zielstrukturen erfolgt hierbei durch visuelle oder automatisierte Feststellung einer oder mehrerer der folgenden Zustände:
- maximierte Rückstreuung des Ziellasers von der Zielstruktur,
- minimierter Durchmesser der Lichtverteilung des Ziellaserstrahles auf der Zielstruktur oder
- charakteristischer Zustand oder Veränderung des OCDR-Signals der Zielstruktur.

Erfindungsgemäß erfolgt die Fokussierung des Kreuzungspunktes der Ziellaser auf die Zielstruktur ZS bevorzugt entweder durch Minimieren des Abstandes mehrerer Ziellaserstrahlen durch den Bediener oder ein Kamerasystem oder auch durch Maximieren der Rückstreuung aus mindestens einem Ziellaserstrahlfokus über konfokale Detektion. Hierbei arbeitet der Ziellaser bevorzugt mit sichtbarem Spektralbereich oder im NIR-Bereich.

Einer vorteilhaften Ausgestaltung entsprechend wird als Ziellaser ein cw-Laser mit gleicher oder ähnlicher Fokuslage wie der Laserstrahl des Laserbehandlungssystems verwendet oder ein cw-Laser mit bekannter bzw. kalibrierter begrenzter Abweichung der Fokuslage zum Laserstrahl des Laserbehandlungssystems, die bei der Laser-Vitreolyse berücksichtigt wird. Diese Berücksichtigung kann beispielsweise dann durch eine entsprechend angepasste Anzeige der Fokuslage oder auch durch momentane Fokusverschiebungen des Laserstrahls des Laserbehandlungssystems vor der Laserauslösung erfolgen, beispielsweise durch eine opto-mechanisch (beispielsweise per verschiebbaren Linsen) oder elektro-optisch (beispielsweise per Flüssigkristalllinsen) realisierte Änderungen der Strahldivergenz.

Als Zielstruktur ZS dienen vorzugsweise die posteriore Seite von Linse (insbesondere einer Intraokularlinse, IOL) oder Kapselsack, die anteriore Retinaoberfläche oder andere Strukturen des Auges.

Es ist aber auch möglich, die Zielstruktur ZS durch den Laserstrahl des Laserbehandlungssystems selbst zu erzeugen. Beispielsweise bewirkt die durch einen Laserschuss des Laserstrahls des Laserbehandlungssystems erzeugte Zielstruktur ZS eine Veränderung im Glaskörper, die wiederum im OCDR eine Signaländerung, wie eine veränderte Rückstreuung oder eine Specklekornveränderung, bewirkt.

Die durch einen Laserschuss des Laserstrahls des Laserbehandlungssystems erzeugte Zielstruktur ZS ist dabei temporär bzw. veränderlich, beispielsweise eine durch mindestens einen Laserschuss erzeugte Gasblase oder eine infolge von Erwärmung temporär veränderte Specklestruktur im OCDR. Solche Veränderungen können auch mittels des Zielaserstrahlls erzeugt werden, beispielsweise, wenn er moduliert wird, und durch Lichtabsorption damit eine lokale charakteristische Signalfluktuation (beispielsweise Specklevariation) im OCDR-Tiefenprofil dort generiert, wo er den OCDR-Strahl schneidet.

Die Erzeugung einer Zielstruktur ZS durch einen Laserschuss des Laserstrahls des Laserbehandlungssystems hat allerdings den Vorteil, dass diese neben der Bestimmung der Fokuslagen auch zur Titration der Laserleistung genutzt werden kann. Diese Titration wäre indirekt über das Absorptionsverhalten des Ziellasers zwar auch möglich, allerdings schwieriger, da ggf. Wellenlängenunterschiede zu berücksichtigen oder zu vermeiden wären.

Erfindungsgemäß werden als Referenzstruktur RS für das OCDR-System die Vorder- oder Rückseite eines vorhandenen Kontaktglases KG, eine im Kontaktglas befindliche technische Struktur, oder aber Augenstrukturen, wie Vorder- oder Rückseite von Hornhaut, Linse, Kapselsack oder die Retinaoberfläche genutzt.

Ist für das ophthalmologische System zur intraokularen Laserbehandlung ein Kontaktglas KG erforderlich, kann die als Referenzstruktur RS realisierte technische Struktur vorzugsweise so ausgebildet sein, dass ein im OCDR charakteristisches Signal erzeugt wird, wie beispielsweise ein Signal mit einem bestimmten Signallevel, Plateau, Verlauf, Position, Abstand oder Mehrfachpeaks oder einer charakteristischen Polarisationsabhängigkeit. Insbesondere könnte die im Kontaktglas KG realisierte Referenzstruktur RS veränderbar, insbesondere schaltbar oder modulierbar sein, beispielsweise über eine Veränderung der Streuung oder Polarisation. Dies könnte zum Beispiel über eine elektrisch geschaltete Flüssigkristallschicht realisiert werden.

Einer weiteren vorteilhaften Ausgestaltung entsprechend wirkt die im Kontaktglas KG realisierte Referenzstruktur RS in einem nichtsichtbaren Spektralband und wird beispielsweise durch ein dielektrisches Reflexionsschichtsystem realisiert.

Dieses dielektrische Reflexionsschichtsystem ist vorzugsweise als Bandpass-Filter ausgebildet, so dass es beispielsweise die Strahlen eines NIR- Ziellasers mit einer Wellenlänge zwischen 780nm ... 850nm teilreflektiert, aber den Behandlungslaser mit einer Wellenlange von 1064nm, sowie das sichtbare Licht mit Wellenlängen zwischen 400nm ... 700nm überwiegend transmittiert. Bei der OCDR-Wellenlänge, beispielsweise 1060nm, wird allerdings durch die Referenzstruktur RZ eine Rückstreuung von maximal 3%, bevorzugt <0.5%, realisiert, um Übersteuerungen im OCDR-Signal zu vermeiden. Bevorzugt ist die Referenzstruktur RS weiter so gestaltet, dass sie im OCDR ein Signal mit einem Signal-Rausch-Abstand, insbesondere zum vom Schrotrauschen verursachten Rauschuntergrund, von über 10dB, über 20dB oder über 30dB aufweist, allerdings auch bevorzugt maximal 40dB.

Die Erkennung der Positionen von Zielstrukturen PZSₙ und der Referenzstruktur PRS wird beispielsweise durch Bestimmung des Maximalwertes oder des Schwerpunktwertes oder eines Schwellwertes des OCDR-Signals oder auch durch Anfitten eines Signalmodels durchgeführt. Die Zuordnung einer so bestimmen Position eines Signals im OCDR zu einer Ziel- oder Referenzstruktur erfolgt dabei bevorzugt durch Verwendung einer erwarteten Signalreihenfolge im OCDR-Signalverlauf (beispielsweise Vorder- oder Rückseite des Kontaktglases, Hornhautoberfläche, ggf. Kapselsackvorderseite, IOL-Vorder- und Rückseite, ggf. Kapselsackrückseite, Netzhautoberfläche). Dabei können auch charakteristische Signalstärken, beispielsweise am Kontaktglas oder der IOL, zum automatisierten Ein- oder Ausschluss von in der Signalreihenfolge erwarteten Strukturen dienen. Auch charakteristische Signalverläufe können dazu genutzt werden, beispielsweise scharfe Reflexe auf den IOL-Oberflächen bei gleichzeitig gegenüber einer natürlichen Linse geringerer Rückstreuungsstärke im Inneren der IOL, d.h. zwischen den scharfen Oberflächenreflexen. Die charakteristischen Signalverläufe können dabei axial, aber auch lateral verlaufen. Beispielsweise können Kapselsackverläufe lateral wesentlich "welliger" verlaufen als die Oberflächen klassischer IOLs. Insbesondere Multifokal-IOLs (z.B. Fresneloptiken) können sogar typische, wiedererkennbare Muster aufweisen. Weiterhin können Plausibilitätschecks für mögliche oder wahrscheinliche Tiefenlagebereiche bestimmter Strukturen genutzt werden, z.B. über wahrscheinliche Hornhautdicken-, Vorderkammertiefen- oder auch Augenlängenbereiche. Des Weiteren können auch vom Bediener gemachte Angaben zur Augenstruktur verwendet werden, beispielsweise im Spezialfall der Verwendung phaker I-OLs.

Einer weiteren vorteilhaften Ausgestaltung entsprechend wird das Bildgebungssystem zusammen mit dem Laserstrahl des Laserbehandlungssystems auf die Zielstrukturen ZS fokussiert, wofür ein Autofokussystem verwendet wird. Bevorzugt weisen das Bildgebungssystem und der Laserstrahl des Laserbehandlungssystems eine NA auf, die sich um einen Faktor <2 unterscheidet.

Nachfolgend wird das Verfahren zur Rekalibrierung des Fokus eines ophthalmologischen Systems zur intraokularen Laserbehandlung anhand eines Behandlungssystems zur Laser-Vitreolyse näher beschrieben.

Das Behandlungssystem zur Laser-Vitreolyse verfügt neben einer Fokussiereinheit und einer Steuereinheit ebenfalls über ein OCDR-System.

Für eine Laser-Vitreolyse sind in Abhängigkeit von der Bearbeitungstiefe ausreichend große Pupillendurchmesser zu gewährleisten. Im Detail sind für eine Laser-Vitreolyse-Behandlung im anterioren Bereich ein Pupillendurchmesser >4mm, im zentralen Bereich >5mm und im posterioren Bereich >6mm günstig. Insbesondere kann die Überprüfung des Pupillendurchmessers dazu verwendet werden, dass der Behandlungslaser nur aktiviert werden kann, wenn ein ausreichend großer Pupillendurchmesser für die jeweilige angestrebte Bearbeitungstiefe erkannt wurde.

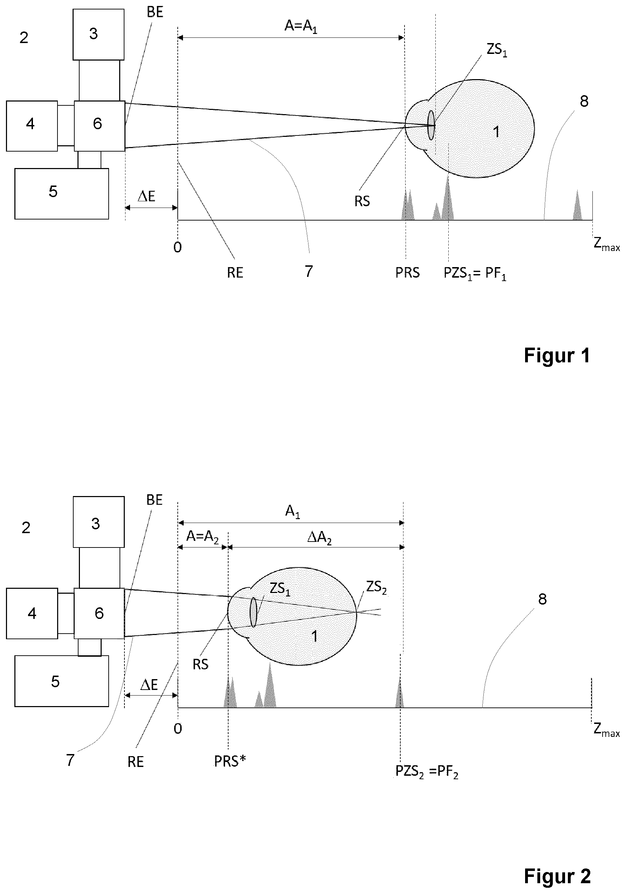

Hierzu zeigt die **Figur 1** das auf eine erste Zielstruktur **ZS₁** im zu behandelnden Auge **1** fokussierte Behandlungssystem zur Laser-Vitreolyse **2.**

Das Behandlungssystem zur Laser-Vitreolyse **2** verfügt über einen Behandlungslaser **3,** ein OCDR-System **4,** ein Bildgebungssystem **5,** ein optisches System **6** und eine (nicht dargestellte) Steuereinheit.

Der Laserstrahl **7** des Behandlungslasers **3** wird auf eine Zielstruktur **ZS₁** im zu behandelnden Auge **1** fokussiert, indem der Abstand **A** des Behandlungssystems zur Laser-Vitreolyse **2** in Bezug auf das Auge **1,** repräsentiert durch eine Referenzstruktur **RS** (beispielsweise die Hornhautoberfläche), ausgehend von einer Bezugsebene **RE** verändert wird, bis bei einem Augenabstand **A=A₁** die Fokussierung des Laserstrahls auf die Zielstruktur **ZS₁** erkennbar ist (hier beispielhaft die Linsenrückseite). Die Erkennung der Fokussierung auf **ZS₁** kann dabei durch Beobachtung einer maximierten Rückstreuung des (abgeschwächten) Laserstrahls **7** oder eines Ziellasers (nicht dargestellt) von der Zielstruktur **ZS1** per Bildgebungssystem **5** erfolgen oder, falls Fokuslage von OCDR-Strahl (nicht dargestellt) und Laserstrahl **7** ausreichend aufeinander abgestimmt sind, an einer Maximierung des OCDR-Signals der Zielstruktur (graue Spitze) an der Position **PZS₁** im OCDR-Signalprofil **8** erfolgen. Das OCDR-Signalprofil **8** erstreckt sich insgesamt über einen relativen Tiefenbereich von 0 bis **Zₘₐₓ** (optischer Weg). Bei einer Fokussierung auf die Zielstruktur **ZS₁** entspricht die Fokuslage **PF₁** des Laserstrahls **7** im OCDR dann genau der OCDR-Position **PZS₁** der Zielstruktur.

Als weitere Bezugsebene **BE** dient hierbei beispielsweise die Linsenfrontfläche des optischen Systems **6.** Da der Messbereich des OCDR-Systems **4** in der Regel nur etwas mehr als die Gesamtlänge des zu behandelnden Auges **1** umfasst, wird für die Messungen über den Referenzarm des OCDR-Systems **4** eine Referenzebene **RE** im Abstand **ΔE** von **BE** eingestellt. Für die Fokusrekalibrierung wird ohne Beschränkung der Allgemeinheit angenommen, dass die Referenzebene zwischen den Kalibrierungsschritten nicht verändert wird. Falls doch, sind die Positionen PZSₙ entsprechend anzupassen. Dabei ist zu beachten, dass die Brechzahl zwischen **BE** und **RE** derjenigen des umgebenden Mediums entspricht (i.A. Luft mit Brechzahl 1). Zwischen **RE** und **RS** kann die Brechzahl diejenige von Luft sein, oder es können im Fall der Verwendung eines Kontaktglases Teilstrecken auch Glas- oder Kunststoffbrechzahlen (beispielsweise n=1,2 ... 1,8) aufweisen. Da zudem die Brechzahlen im Auge etwas unterschiedlich sind (Kammerwasser und Glaskörper ca. 1,36 und Hornhaut ca. 1,38, IOL abhängig vom verwendeten Material) und auch von Patient zu Patient variieren können, empfiehlt es sich, generell Positionen PZSₙ und auch die näherungsweise zu bestimmenden Fokusposition PF(ΔA) jeweils als optische Weglängen gegenüber der Referenzebene RE zu bestimmen. Um im Verlauf der Fokusrekalibrierung möglichst wenige oder keine Anpassungen von ΔE vornehmen zu müssen, wird der vom OCDR-Signalprofil 8 abzudeckende Tiefenbereich 0 bis Zₘₐₓ so gewählt, dass mindestens die Tiefe des posterioren Augenabschnitts (>25 mm optischer Weg) abgedeckt wird, möglichst aber die gesamte durchschnittliche Augenlänge (>34mm optisch) oder idealerweise aber ein erweiterter Bereich von >60 mm oder >100mm (jeweils optisch). Dazu sind OCDR-Systeme mit entsprechender Kohärenzlänge zu verwenden.

Danach werden der Abstand **A₁** und die Zielstrukturposition **PZS₁** aus dem OCDR-Signalprofil **8** jeweils als optische Weglängen gegenüber der Referenzebene **RE** bestimmt. Hierbei wird beispielhaft als Referenzstruktur **RS** die Vorderfläche der Hornhaut und als Zielstruktur **ZS₁** hier die Linsenrückseite verwendet.

Im OCDR-Signalprofil **8** entsprechen die schematisch dargestellten Signalspitzen von links (0) nach rechts (dem maximalen Messbereich **Zₘₐₓ**) der Vorder- und Rückfläche der Hornhaut, der Vorder- und Rückfläche der Linse und der Retinaoberfläche des zu behandelnden Auges **1.** Der Rauschuntergrund, Signale von tieferen Retina- oder Choroidschichten und die Kapselsacksignale wurden der Übersichtlichkeit halber hier nicht dargestellt. Ob die Kapselsackvon den Linsenoberflächensignalen unterscheidbar sind, hängt beispielsweise von der speziellen Situation ab, d.h. ob der Kapselsack an der Linse anliegt oder nicht und auch von der Empfindlichkeit und dem Auflösungsvermögen des OCDR-Systems.

Die **Figur 2** zeigt das auf eine zweite Zielstruktur **ZS₂** im zu behandelnden Auge **1** fokussierte Behandlungssystem zur Laser-Vitreolyse **2.**

Der Laserstrahl **7** des Behandlungslasers **3** wird dazu auf eine Zielstruktur **ZS₂** im zu behandelnden Auge **1** fokussiert, indem der Abstand **A** des Behandlungssystems zur Laser-Vitreolyse **2** in Bezug auf das Auge **1** ausgehend von einer Bezugsebene **RE** gegenüber der Ausgangsposition **A₁** (um ΔA) so verändert wird, bis die Fokussierung des Laserstrahls auf die jeweiligen Zielstruktur **ZS₂** wiederum erkennbar ist (wie erwähnt, beispielsweise anhand maximierter Behandlungs- oder Ziellaserrückstreuung oder ggf. lokaler OCDR-Signalmaximierung bei abgestimmter Strahlgeometrie zwischen OCDR und Behandlungslaser). Dazu kann das Behandlungssystems zur Laser-Vitreolyse **2** in Bezug auf das Auge **1** bewegt werden (per nicht dargestellter manuell oder motorisch bewegter Gerätebasis) oder auch umgekehrt (beispielsweise per motorisierter Patientenkopfstütze).

Danach werden der Abstand **A=A₂** bzw. **ΔA₂=A₁-A₂** und die Position des OCDR-Signals **PZS₂** der zweiten Zielstruktur **ZS₂** aus dem OCDR-Signalprofil 8 bestimmt, wobei hier als Referenzstruktur **RS** weiterhin die Vorderfläche der Hornhaut und als zweite Zielstruktur **ZS₂** die Retinaoberfläche dient. Die Position der Referenzstruktur im OCDR-Signalprofil 8 ist nun **PRS*,** die anders ist als die Position der Referenzstruktur **PRS** im Falle der Fokussierung auf ZS₁.

Wegen der Fokussierung auf die zweite Zielstruktur **ZS₂** entspricht nun die Position der Zielstruktur **PZS₂** auch der Position **PF₂** des Fokus des Behandlungslasers **3,** jeweils wiederum als optische Wege bezogen auf die Referenzebene **RE.**

Aus den so ermittelten Werten A₁, ΔA₂, PZS₁, PZS₂ lässt sich nun mindestens eine Funktion $PF \left(ΔA\right) = {f}_{{A}_{1} , {ΔA}_{2} , {PZS}_{1} , {PZS}_{2} ,} \left(ΔA\right)$ ableiten, die idealerweise für $PF \left({ΔA}_{1}=0\right) = {PZS}_{1} und für PF \left({ΔA}_{2}\right) = {PZS}_{2}$ ergibt und mit deren Hilfe näherungsweise für andere, beliebig wählbare Werte einer Abstandsveränderung **ΔA** die jeweils dafür anzunehmende Fokusposition **PF** des Behandlungslasers **3** näherungsweise bestimmt werden kann. Die Funktion PF(ΔA) soll dabei möglichst eine Abweichung zwischen näherungsweise bestimmter und tatsächlicher Fokusposition des Behandlungslasers von weniger als 2 Rayleigh-Längen des Behandlungslaserfokus aufweisen, insbesondere weniger als 1 Rayleigh-Länge oder besonders bevorzugt, weniger als 0,5 der Rayleigh-Länge.

Der in **Figur 2** dargestellte Schritt der Bestimmung der Parameter ΔA₂ und PZS₂ lässt sich nun analog für weitere Schritte von Fokussierungen auf Zielstrukturen ZS₃ ..ZS_{N} wiederholen, um letztendlich einen erweiterten Satz von Parametern A₁, ΔA₂ ... ΔA*_{N}, PZS*₁ ... *PZS_{N}* zu gewinnen, über den dann eine Funktion PF(ΔA)= *fA*_{1,}Δ*A*_{2 *..*. ΔA*_{N,PZS}*1.*.*. *PZSN*}(Δ*A*) mit noch erhöhter Genauigkeit der Abschätzung der Fokusposition des Behandlungslasers für beliebige Augenabstände ΔA bestimmt werden kann. Auch hierbei soll die ermittelte Funktion idealerweise genau durch die Messpunkte verlaufen, d.h. PF(ΔAₙ)=PZSₙ, aber im gesamten Verlauf bevorzugt immer eine Abweichung zwischen näherungsweise bestimmter und tatsächlicher Behandlungslaserfokusposition von weniger als 2 Rayleigh-Längen des Behandlungslaserfokus ermöglichen, insbesondere weniger als 1 Rayleigh-Länge oder besonders bevorzugt, weniger als 0,5 der Rayleigh-Länge.

Da insbesondere auch bei der Laser-Vitreolyse-Behandlung Kontaktgläser zum Einsatz kommen, wird im Folgenden darauf näher eingegangen.

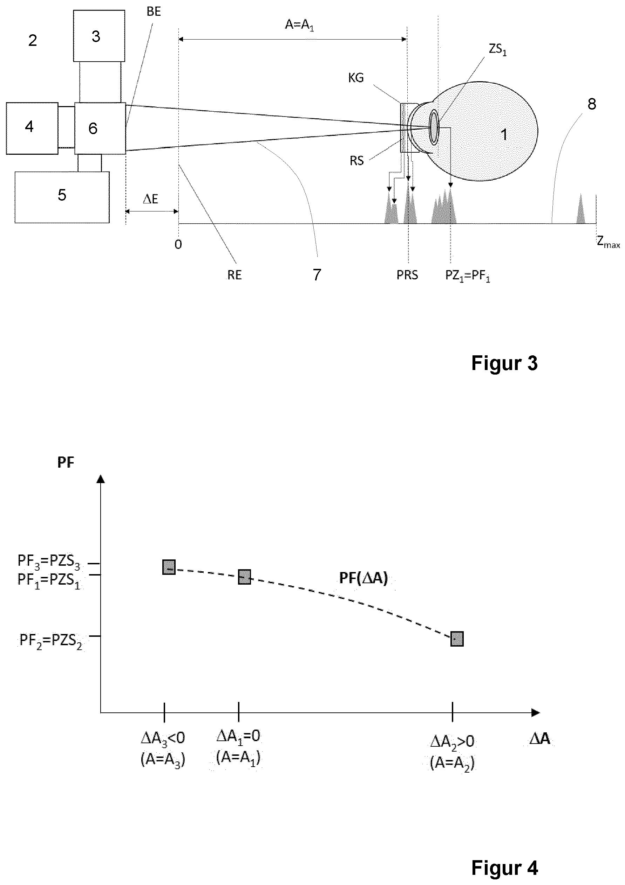

Hierzu zeigt die **Figur 3** ein Behandlungssystem zur Laser-Vitreolyse, welches ein Kontaktglas **KG** mit einer Referenzstruktur **RS** nutzt.

Das Behandlungssystem zur Laser-Vitreolyse **2** verfügt über einen Behandlungslaser **3,** ein OCDR-System **4,** ein Bildgebungssystem **5,** ein optisches System **6,** eine (nicht dargestellte) Steuereinheit und sieht die Verwendung eines Kontaktglases **KG** vor.

Der Laserstrahl **7** des Behandlungslasers **3** wird auf eine Zielstruktur **ZS₁** (in diesem Fall die Kapselsackvorderseite) im zu behandelnden Auge **1** fokussiert, indem der Abstand **A des** Behandlungssystems zur Laser-Vitreolyse **2** ausgehend von einer Bezugsebene **RE** in Bezug auf das Auge **1** verändert wird, bis die Fokussierung des Laserstrahls **7** auf die jeweilige Zielstruktur **ZS₁** erkennbar ist (wie erwähnt, beispielsweise anhand maximierter Behandlungs- oder Ziellaserrückstreuung oder ggf. lokaler OCDR-Signalmaximierung bei abgestimmter Strahlgeometrie zwischen OCDR und Behandlungslaser).

Danach werden der Abstand **A=A₁** von der Referenzebene **RE** zur Position der Referenzstruktur **PRS,** sowie die Zielstrukturposition **PZS₁** aus dem OCDR-Signalprofil **8** jeweils als optische Weglängen gegenüber der Referenzebene **RE** bestimmt. Hierbei befindet sich in der hier gezeigten Ausführungsvariante die Referenzstruktur **RS** in dem verwendeten Kontaktglas **KG.** Die Referenzstruktur befindet sich also außerhalb des Auges, hat jedoch durch den Kontakt einen ausreichend festen Bezug zum Auge.

Wegen der Fokussierung auf die Zielstruktur **ZS₁** entspricht somit die Position der Zielstruktur **PZS₁** auch der Position **PF₁** des Fokus des Behandlungslasers **3.**

Im OCDR-Signalprofil **8** entsprechen die dargestellten Signalspitzen von links **(0)** nach rechts (dem maximalen Messbereich **Zₘₐₓ**) der Vorderfläche des Kontaktglases, der technischen Struktur **RS** im Kontaktglas, der Vorder- und Rückfläche der Hornhaut, der Vorderfläche des Kapselsacks, der Vorderfläche und Rückfläche der Linse, der Rückseite des Kapselsacks und der Retina des zu behandelnden Auges **1.**

Wie oben beschrieben kann als Referenzstruktur RS die Vorder- oder Rückseite eines vorhandenen Kontaktglases KG oder eine im Kontaktglas befindliche technische Struktur genutzt werden, die noch individuell veränderbar, insbesondere schaltbar oder modulierbar ausgebildet sein kann.

In der **Figur 4** ist beispielhaft die Funktion **PF(ΔA)** zur Bestimmung beliebiger Fokuspositionen **PF** des Laserstrahls des Laserbehandlungssystems dargestellt. Der Ziellaserstrahl des Laserbehandlungssystems wurde hier auf drei Zielstrukturen **ZS₁, ZS₂** und **ZS₃** im zu behandelnden Auge fokussiert, indem der Abstand **A** des Laserbehandlungssystems zum Auge verändert wurde, bis die Fokussierung des Ziellaserstrahls des Laserbehandlungssystems auf die Zielstrukturen **ZS₁, ZS₂** und **ZS₃** detektiert und die entsprechenden Abstände **A₁, A₂** und **A₃** bzw. Abstandsveränderungen **ΔA₂** und **ΔA₃** gegenüber **A₁** bestimmt wurden. Anhand der sich aus diesen Stützstellen ergebenden Funktion **PF(ΔA)** lässt sich für beliebig wählbare Werte einer Abstandsveränderung **ΔA** des Laserbehandlungssystems zum Auge die jeweils anzunehmende Fokusposition **PF** des Laserstrahls des Laserbehandlungssystems bestimmen.

Einer weiteren bevorzugten Ausgestaltung entsprechend wird das Bildgebungssystem zusammen mit dem Laser des Vitreolyse-Systems bewegt und die Fokussierung auf die Zielstrukturen ZS jeweils mittels eines Autofokussystems realisiert. Hierbei erfolgt die Fokussierung beispielsweise durch Brennweitenänderung oder Abstandsveränderung zwischen System und Patientenauge, wozu beispielsweise auch eine motorisierte Kopfstütze oder ein motorisierter Gerätekopf Verwendung finden können.

Hierbei ist es von Vorteil, wenn das Bildgebungssystem und der Behandlungslaser eine ähnliche numerische Apertur (NA) aufweisen, d.h. die sich um einen Faktor <2 unterscheiden.

Weiterhin ist von Vorteil, wenn das OCDR-System und der Behandlungslaser mit ähnlichen Wellenlängen, d.h. mit einer Abweichung <10% arbeiten. Bevorzugt werden dabei Wellenlängen um 1060nm verwendet, da hier langkohärente, durchstimmbare Laser für das OCDR genutzt werden können (d.h. SS-OCDRs), sowie YAG-Laser bei 1064nm als Behandlungslaser.

Die vorgeschlagene Anordnung zur Rekalibrierung des Fokus eines ophthalmologischen Systems zur intraokularen Laserbehandlung besteht aus einer Behandlungslasereinheit, einer Bildgebungseinheit und einem optischen System zur Fokussierung und Strahlüberlagerung, sowie einem OCDR-System und einer Steuereinheit.

Für die Beschreibung der Funktion der Anordnung zur Rekalibrierung des Fokus eines ophthalmologischen Systems zur intraokularen Laserbehandlung wird auf das zuvor beschriebene Verfahren verwiesen.

Erfindungsgemäß ist das Laserbehandlungssystem so ausgebildet, dass der Abstand A zum Auge veränderbar und ein Ziellaserstrahl auf mindestens eine Zielstruktur ZS₁ im zu behandelnden Auge fokussierbar ist. Die Steuereinheit ist ausgebildet, einen Abstand A₁ aus der Position einer gewählten Referenzstruktur PRS, sowie die Position der Zielstruktur PZS₁ im OCDR-Signalprofil jeweils bezogen auf eine Referenzebene RE zu bestimmen und für beliebig wählbare Werte einer Abstandsveränderung ΔA des Laserbehandlungssystems zum

Auge, unter Verwendung der Parameter A₁ und PZS₁ eine Funktion $PF \left(ΔA\right) = {f}_{{A}_{1} , {PZS}_{1}} \left(ΔA\right)$ zu bestimmen und die jeweils anzunehmende Fokusposition **PF** des Laserstrahls des Laserbehandlungssystems im OCDR-Signalprofil näherungsweise zu berechnen.

Bevorzugt ist das Laserbehandlungssystem so ausgebildet, dass der Ziellaserstrahl außer auf ZS₁ schrittweise auf N-1 weitere Zielstrukturen ZS₂ ... ZS_{N} fokussierbar ist. Die Steuereinheit ist ausgebildet, im OCDR-Signalprofil dafür die jeweiligen Positionen der Zielstrukturen PZS₂ ... PZS_{N} und die jeweiligen Änderungen ΔA₂ ... ΔA_{N} der Position der Referenzstruktur gegenüber ihrer Anfangsposition PRS=A₁ bei der Fokussierung auf die erste Zielstruktur ZS1 zu bestimmen und aus den Parametern A₁, ΔA₂ ... ΔA*_{N}, PZS*₁ ... *PZS_{N}* dann eine Funktion *PF*(Δ*A*) *= f*_{*A*₁,Δ*A*₂*...* ΔA_{*N,PZS*1}*... PZSN*}(Δ*A*) zu bestimmen, aus der für beliebig wählbare Werte einer Abstandsveränderung ΔA des Laserbehandlungssystems zum Auge die jeweils anzunehmende Fokusposition PF des Laserstrahls des Laserbehandlungssystems im OCDR-Signalprofil näherungsweise berechenbar ist.

Eine erste Gruppe vorteilhafter Ausgestaltungen betreffen das Laserbehandlungssystem. So ist es beispielsweise möglich, dass als Ziellaserstrahl der Behandlungslaserstrahl des Laserbehandlungssystems mit reduzierter Pulsenergie, welche keine Photodisruption auslösen kann, nutzbar ist. Es ist aber auch möglich, dass als Ziellaserstrahl ein zusätzlicher Laserstrahl genutzt wird, dessen Parameter keine permanente Gewebsveränderung im Auge zulassen.

Es kann weiterhin vorgesehen sein, dass das Laserbehandlungssystem über mehrere Ziellaser verfügt, die sich am Ort des Fokus des Behandlungslasers des Laserbehandlungssystems kreuzen und zur Fokussierung auf eine Zielstruktur ZS im zu behandelnden Auge dienen. Dabei weisen die cw-Laserstrahlen des Ziellasers vorzugsweise eine gleiche oder ähnliche Fokuslage auf wie der Laserstrahl des Laserbehandlungssystems.

Einer weiteren vorteilhaften Ausgestaltung entsprechend ist das Laserbehandlungssystem ausgebildet Zielstrukturen ZS im Auge selbst zu erzeugen. Dies kann beispielsweise durch einen Puls oder eine Modulation des Ziellasers erfolgen, die eine Veränderung im Auge bewirken und eine Signaländerung im OCDR, oder eine veränderte Rückstreuung oder eine Phasen- oder Specklekornveränderung im OCDR-Signal bewirken. Diese Zielstrukturen ZS sind temporär bzw. veränderlich, werden beispielsweise durch mindestens einen Laserschuss erzeugt und bewirken die Bildung einer Gasblase oder eine infolge von Temperaturänderung temporär veränderte Specklestruktur im OCDR.

Einer besonders vorteilhaften Ausgestaltung entsprechend ist das Laserbehandlungssystem zur Laser-Vitreolyse ausgebildet und erzeugt durch einen Laserstrahl eine Zielstruktur ZS in der Nähe einer zu bearbeitenden Struktur.

Eine zweite Gruppe vorteilhafter Ausgestaltungen betreffen die Steuereinheit, die ausgebildet ist, die Detektion einer Fokussierung auf eine Zielstruktur durch automatisierte Feststellung einer oder mehrerer der folgenden Zustände festzustellen:
- maximierte Rückstreuung des Ziellasers von der Zielstruktur,
- minimierter Durchmesser der Ziellaserstrahllichtverteilung auf der Zielstruktur und/oder
- charakteristischer Zustand oder charakteristische Veränderung des OCDR-Signals der Zielstruktur.

Die Steuereinheit ist weiter ausgebildet, die durch einen Laserschuss des Laserstrahls des Laserbehandlungssystems erzeugte Zielstruktur ZS neben der Bestimmung der Fokuslagen auch zur Titration der Laserleistung zu nutzen.

Außerdem kann von der Steuereinheit die Positionen von Zielstrukturen und der Referenzstruktur beispielsweise durch Bestimmung des Maximalwertes oder des Schwerpunktwertes oder eines Schwellwertes eines OCDR-Signals im OCDR-Signalprofil erkannt werden.

Für die Funktion *f*_{*A*₁*,*ΔA₂ *...* ΔA}*_{N},*_{*PZS*₁ *... PZSN*}(Δ*A*) zur Bestimmung der Fokuspositionen **PF** wird von der Steuereinheit ein Polynom ersten bis N-ten Grades oder eine andere nichtlineare Funktion mit N Freiheitsgraden genutzt.

Es kann aber auch ein Polynom oder eine nichtlineare Funktion höheren Grades als N genutzt werden. Zur Bestimmung der Funktion f können zusätzlich anderweitig ermittelte Parameter des Kontaktglases, wie Krümmungsradien, Dicken oder Brechzahlen, oder zusätzliche anderweitig ermittelte Parameter des Auges, wie Brechzahlen, Dicken oder Radien von Hornhaut oder Linse herangezogen werden.

Insbesondere ist die Steuereinheit so ausgebildet, dass die Abweichung der näherungsweisen Bestimmung der anzunehmenden Fokusposition **PF** des Laserstrahls weniger als 2 Rayleigh-Längen des Laserfokus, insbesondere weniger als 1 Rayleigh-Länge oder besonders bevorzugt, weniger als 0,5 der Rayleigh-Länge beträgt.

Eine dritte Gruppe vorteilhafter Ausgestaltungen betreffen ein zusätzlich vorhandenes Kamerasystem, welches die Ziellaser und die Zielstruktur ZS erfasst. Aus diesen Aufnahmen wird von der Steuereinheit die Fokussierung zum Beispiel durch
- Minimieren des Abstandes von Zielstrahllaserpositionen,
- Minimieren der Ortsvariation eines sich periodisch bewegenden Ziellaserstrahls oder
- Maximieren der Rückstreuung aus mindestens einem Zielstrahllaserfokus bestimmt.

Eine vierte Gruppe vorteilhafter Ausgestaltungen betreffen ein zusätzlich verwendetes Kontaktglas, dessen Vorder- oder Rückseite oder eine im Kontaktglas befindliche technische Struktur als Referenzstruktur RS dient.

Dabei erzeugt die im Kontaktglas KG als Referenzstruktur RS realisierte technische Struktur ein im OCDR charakteristisches Signal, mit einem bestimmten Level, Plateau, Verlauf, Position, Abstand oder Mehrfachpeaks oder eine charakteristische Polarisationsabhängigkeit des Signals. Die realisierte Referenzstruktur RS ist vorzugsweise veränderbar, insbesondere schaltbar oder modulierbar, wie beispielsweise über eine Veränderung der Streuung oder Polarisation.

Besonders bevorzugt wirkt die im Kontaktglas KG realisierte Referenzstruktur RS in einem nichtsichtbaren Spektralband und wird beispielsweise durch eine dielektrisches Reflexionsschichtsystem realisiert. Das dielektrische Reflexionsschichtsystem ist insbesondere derart ausgebildet, dass die Ziellaserstrahlen bei einer Wellenlänge zwischen 400nm ... 1050nm reflektiert und der Behandlungslaserstrahl des Laserbehandlungssystems bei Wellenlängen >1050nm überwiegend transmittiert werden.

Eine fünfte Gruppe vorteilhafter Ausgestaltungen betreffen ein vorhandenes Bildgebungssystem, welches zum Beispiel über ein Autofokussystem zusammen mit dem Laserstrahl des Laserbehandlungssystems auf die Zielstrukturen ZS fokussiert wird.

Vorzugsweise weisen das Bildgebungssystem und der Laserstrahl des Laserbehandlungssystems ein NA auf, die sich um einen Faktor <2 unterscheiden.

Das Bildgebungssystem und der Laserstrahl des Laserbehandlungssystems können aber auch einen bestimmten Fokussierungsunterschied aufweisen, um unterschiedliche Wellenlängen zu kompensieren.

Vorzugsweise ist das Bildgebungssystem ausgebildet, einen in Abhängigkeit von der Bearbeitungstiefe ausreichend großen Pupillendurchmesser zu sichern.

Einer besonders vorteilhaften Ausgestaltung entsprechend ist das Bildgebungssystem ausgebildet, die über die Fokusrekalibrierung abgeschätzte Fokuslage in Bezug auf Augenstrukturen vor der Behandlungslaseraktivierung darzustellen.

Eine letzte Gruppe vorteilhafter Ausgestaltungen betreffen das OCDR-System. Das OCDR-System und der Laserstrahl des Laserbehandlungssystems weisen bevorzugt eine NA auf, die sich um einen Faktor <2 unterscheiden.

Vorzugsweise weisen das OCDR-System und der Laserstrahl des Laserbehandlungssystems Wellenlängen auf, die höchstens 10% voneinander abweichen.

Es ist aber auch möglich, dass das OCDR-System und der Laserstrahl des Laserbehandlungssystems einen bestimmten Fokussierungsunterschied aufweisen, um unterschiedliche Wellenlängen zu kompensieren.

Mit der erfindungsgemäßen Anordnung wird eine Lösung zur Rekalibrierung des Fokus eines ophthalmologischen Laserbehandlungssystems zur Verfügung gestellt, welche die Nachteile der bekannten technischen Lösungen behebt und die Individualität eines zu behandelnden Auges und der Toleranzen des optischen Systems bei der Rekalibrierung des Fokus des Behandlungslasers berücksichtigt.

Dadurch ist es möglich, die Fokuslage des Behandlungslasers für jede neue Behandlungssituation, und zwar unabhängig vom Wechsel des Patienten und/oder Kontaktglases oder auch einer Veränderung der Brennweite des Behandlungslasers, der Akkommodation und/oder insbesondere der Patientenaugenposition, zu bestimmen.

Die vorgeschlagene Lösung lässt sich zudem leicht implementieren, ist kostengünstig und ermöglicht eine einfachere, schnellere und vor allem sicherere Laserbehandlung am Auge.

Mit der erfindungsgemäßen Lösung lassen sich verlässlich und präzise Sperrbereiche einrichten, in denen eine Laserbehandlung ausgeschlossen werden kann, was insbesondere für Systeme zur Laservitreolyse von Nutzen ist, um empfindliche Augenstrukturen zu schützen und schonen.

Obwohl die vorgeschlagene Lösung insbesondere für Behandlungssysteme zur Laser-Vitreolyse vorgesehen ist, bietet sie, wie oben erwähnt zahlreiche andere intraokuläre Anwendungsmöglichkeiten, die von der vorgeschlagenen Lösung zur Rekalibrierung des Fokus eines Laserbehandlungssystems ebenfalls profitieren würden.

## Patentansprüche

1. Anordnung zur Rekalibrierung des Fokus eines ophthalmologischen Systems zur intraokularen Laserbehandlung bestehend aus einer Behandlungslasereinheit, einer Bildgebungseinheit und einem optischen System zur Fokussierung und Strahlüberlagerung sowie einem OCDR-System und einer Steuereinheit, **dadurch gekennzeichnet, dass** das Laserbehandlungssystem so ausgebildet ist, dass der Abstand A zum Auge veränderbar und ein Ziellaserstrahl auf mindestens eine Zielstruktur ZS₁ im zu behandelnden Auge fokussierbar ist, dass die Steuereinheit ausgebildet ist, einen Abstand A₁ aus der Position einer gewählten Referenzstruktur PRS, sowie die Position der Zielstruktur PZS₁ im OCDR-Signalprofil jeweils bezogen auf eine Referenzebene RE zu bestimmen und dass die Steuereinheit weiter ausgebildet ist, für beliebig wählbare Werte einer Abstandsveränderung ΔA des Laserbehandlungssystems zum Auge die jeweils anzunehmende Fokusposition PF des Laserstrahls des Laserbehandlungssystems im OCDR-Signalprofil näherungsweise unter Verwendung der Parameter A₁ und PZS₁ abzuschätzen.

2. Anordnung nach Anspruch 1, **gekennzeichnet dadurch, dass** die Steuereinheit ausgebildet ist, die ermittelten Werte A₁ und PZS₁ zu verwenden, eine Funktion $PF \left(ΔA\right) = {f}_{{A}_{1} , {PZS}_{1}} \left(ΔA\right)$ zu bestimmen, um für beliebig wählbare Werte einer Abstandsveränderung ΔA des Laserbehandlungssystems zum Auge die jeweils anzunehmende Fokusposition PF des Laserstrahls des Laserbehandlungssystems im OCDR-Signalprofil näherungsweise zu berechnen.

3. Anordnung nach Anspruch 1, **dadurch gekennzeichnet, dass** das Laserbehandlungssystem so ausgebildet ist, dass der Ziellaserstrahl außer auf ZS₁ schrittweise auf N-1 weitere Zielstrukturen ZS₂ ... ZS_{N} fokussierbar ist und dass die Steuereinheit ausgebildet ist, im OCDR-Signalprofil dafür die jeweiligen Positionen der Zielstrukturen PZS₂ ... PZS_{N} und die jeweiligen Änderungen ΔA₂ ... ΔA_{N} der Position der Referenzstruktur gegenüber ihrer Anfangsposition PRS=A₁ bei der Fokussierung auf die erste Zielstruktur ZS1 zu bestimmen und aus den Parametern *A*₁, ΔA₂ ... ΔA*_{N},* **PZS₁** ... *PZS_{N}* dann eine Funktion *PF*(Δ*A*) = f_{*A*₁,ΔA₂... ΔA_{*N,PZS*1}... *PZSN*}(Δ*A*) zu bestimmen, aus der für beliebig wählbare Werte einer Abstandsveränderung ΔA des Laserbehandlungssystems zum Auge die jeweils anzunehmende Fokusposition **PF** des Laserstrahls des Laserbehandlungssystems im OCDR-Signalprofil näherungsweise berechenbar ist.

4. Anordnung nach Anspruch 1, **dadurch gekennzeichnet, dass** das Laserbehandlungssystem so ausgebildet ist, dass als Ziellaserstrahl der Behandlungslaserstrahl des Laserbehandlungssystems mit reduzierter Pulsenergie, welche keine Photodisruption auslösen kann, nutzbar ist.

5. Anordnung nach Anspruch1, 2, 3 oder 4, **dadurch gekennzeichnet, dass** die Steuereinheit ausgebildet ist, die Detektion einer Fokussierung auf eine Zielstruktur durch automatisierte Feststellung einer oder mehrerer der folgenden Zustände festzustellen: 1) maximierte Rückstreuung des Ziellasers von der Zielstruktur 2) minimierter Durchmesser der Ziellaserstrahllichtverteilung auf der Zielstruktur 3) charakteristischer Zustand oder charakteristische Veränderung des OCDR-Signals der Zielstruktur.

6. Anordnung nach Anspruch 1, **dadurch gekennzeichnet, dass** das Laserbehandlungssystem über mehrere Ziellaser verfügt, die sich am Ort des Fokus des Behandlungslasers des Laserbehandlungssystems kreuzen und zur Fokussierung auf eine Zielstruktur ZS im zu behandelnden Auge dienen, wobei ein Kamerasystem vorhanden ist, welches die Fokussierung von Ziellasern auf die Zielstruktur ZS durch Minimieren des Abstandes von Zielstrahllaserpositionen erfasst.

7. Anordnung nach Anspruch 1, **dadurch gekennzeichnet, dass** das Laserbehandlungssystem ausgebildet ist Zielstrukturen ZS im Auge selbst zu erzeugen.

8. Anordnung nach Anspruch 4, **dadurch gekennzeichnet, dass** das Laserbehandlungssystem ausgebildet ist eine Zielstruktur ZS im Auge durch einen Puls oder eine Modulation des Ziellasers zu erzeugen, die eine Veränderung im Auge ist, die eine Signaländerung im OCDR, oder eine veränderte Rückstreuung oder eine Phasen- oder Specklekornveränderung im OCDR-Signal bewirkt.

9. Anordnung nach Anspruch 1, **dadurch gekennzeichnet, dass** das Laserbehandlungssystem ausgebildet ist eine Zielstruktur ZS im Auge zu erzeugen, die temporär bzw. veränderlich ist, beispielsweise eine durch mindestens einen Laserschuss erzeugte Gasblase oder eine infolge von Temperaturänderung temporär veränderte Specklestruktur im OCDR ist.

10. Anordnung nach Anspruch 1, **dadurch gekennzeichnet, dass** die Steuereinheit ausgebildet ist, die durch einen Laserschuss des Laserstrahls des Laserbehandlungssystems erzeugte Zielstruktur ZS neben der Bestimmung der Fokuslagen auch zur Titration der Laserleistung zu nutzen.

11. Anordnung nach Anspruch 1, **dadurch gekennzeichnet, dass** das dielektrische Reflexionsschichtsystem derart ausgebildet ist, dass die Ziellaserstrahlen bei einer Wellenlänge zwischen 400nm ... 1050nm reflektiert und der Behandlungslaserstrahl des Laserbehandlungssystems bei Wellenlängen >1050nm überwiegend transmittiert werden.

12. Anordnung nach Anspruch 1, **dadurch gekennzeichnet, dass** das Bildgebungssystem und der Laserstrahl des Laserbehandlungssystems ein NA aufweisen, die sich um einen Faktor <2 unterscheiden.

13. Anordnung nach Anspruch 1, **dadurch gekennzeichnet, dass** das OCDR-system und der Laserstrahl des Laserbehandlungssystems ein **NA** aufweisen, die sich um einen Faktor <2 unterscheiden.

14. Anordnung nach Anspruch 1, **dadurch gekennzeichnet, dass** das OCDR-System und der Laserstrahl des Laserbehandlungssystems Wellenlängen aufweisen, die höchstens 10% voneinander abweichen.

15. Anordnung nach Anspruch 1, **dadurch gekennzeichnet, dass** die Steuereinheit so ausgebildet ist, dass die Abweichung der näherungsweisen Bestimmung der anzunehmenden Fokusposition **PF** des Laserstrahls weniger als 2 Rayleigh-Längen des Laserfokus, insbesondere weniger als 1 Rayleigh-Länge oder besonders bevorzugt, weniger als 0,5 der Rayleigh-Länge beträgt.

## Claims

1. Arrangement for recalibrating the focus of an ophthalmological system for intraocular laser treatment, consisting of a treatment laser unit, an imaging unit and an optical system for focusing and beam superposition, and an OCDR system and a control unit, **characterized in that** the laser treatment system is designed such that the distance A to the eye is alterable and a target laser beam is focusable on at least one target structure ZS₁ in the eye to be treated, **in that** the control unit is designed to determine a distance A₁ from the position of a chosen reference structure PRS, and the position of the target structure PZS₁ in the OCDR signal profile, in each case in relation to a reference plane RE, and **in that** the control unit is further designed, for any selectable values of an alteration in distance ΔA of the laser treatment system from the eye, to approximately estimate the respective assumable focus position PF of the laser beam of the laser treatment system in the OCDR signal profile using the parameter A₁ and PZS₁.

2. Arrangement according to Claim 1, **characterized in that** the control unit is designed to use the ascertained values A₁ and PZS₁ to determine a function $PF \left(ΔA\right) = {f}_{{A}_{1} , PZ {S}_{1}} \left(ΔA\right)$ in order, for any selectable values of an alteration in distance ΔA of the laser treatment system from the eye, to approximately calculate the respective assumable focus position PF of the laser beam of the laser treatment system in the OCDR signal profile.

3. Arrangement according to Claim 1, **characterized in that** the laser treatment system is designed so that, apart from ZS₁, the target laser beam is incrementally focusable on N-1 further target structures ZS₂, ..., ZS_{N} and **in that**, to this end, the control unit is designed to determine, in the OCDR signal profile, the respective positions of the target structures PZS₂, ..., PZS_{N} and the respective alterations ΔA₂, ..., ΔA_{N} in the position of the reference structure vis-à-vis its initial position PRS = A₁ when focusing on the first target structure ZS1 and then, using the parameters *A*₁,*ΔA*₂ ... *Δ*A*_{N},PZS*₁ ... *PZS_{M},* to determine a function *PF*(*ΔA*) = *f*_{*A*₁,*MA*₁, ... *MAN*},*PZS*ₙ ... *DZS_{n(ΔA}*), from which, for any selectable values of an alteration in distance ΔA of the laser treatment system from the eye, the respective assumable focus position PF of the laser beam of the laser treatment system in the OCDR signal profile is approximately calculable.

4. Arrangement according to Claim 1, **characterized in that** the laser treatment system is designed such that the treatment laser beam of the laser treatment system with a reduced pulse energy, which cannot trigger photodisruption, is usable as target laser beam.

5. Arrangement according to Claim 1, 2, 3 or 4, **characterized in that** the control unit is designed to establish the detection of focusing on a target structure by automated establishment of one or more of the following states: 1) maximized backscatter of the target laser from the target structure, 2) minimized diameter of the target laser beam light distribution on the target structure, 3) characteristic state or characteristic alteration in the OCDR signal of the target structure.

6. Arrangement according to Claim 1, **characterized in that** the laser treatment system comprises a plurality of target lasers which intersect at the location of the focus of the treatment laser of the laser treatment system and which serve to focus on a target structure ZS in the eye to be treated, wherein a camera system is present and detects the focusing of target lasers on the target structure ZS by minimizing the spacing of target beam laser positions.

7. Arrangement according to Claim 1, **characterized in that** the laser treatment system itself is designed to create target structures ZS in the eye.

8. Arrangement according to Claim 4, **characterized in that**
the laser treatment system is designed to create a target structure ZS in the eye by a pulse or a modulation of the target laser, said target structure being an alteration in the eye, which brings about a signal alteration in the OCDR or a altered backscatter or a phase or speckle grain alteration in the OCDR signal.

9. Arrangement according to Claim 1, **characterized in that** the laser treatment system is designed to create a temporary or alterable target structure ZS in the eye, for example a gas bubble created by at least one laser shot or a speckle structure in the OCDR that has been temporarily altered as a consequence of temperature alterations.

10. Arrangement according to Claim 1, **characterized in that** the control unit is designed to use the target structure ZS created by a laser shot of the laser beam of the laser treatment system not only to determine the focal positions but also to titrate the laser power.

11. Arrangement according to Claim 1, **characterized in that** the dielectric reflection layer system is designed in such a way that the target laser beams at a wavelength of between 400 nm ... 1050 nm are reflected and the treatment laser beam of the laser treatment system at wavelengths >1050 nm is predominantly transmitted.

12. Arrangement according to Claim 1, **characterized in that** an NA of the imaging system and an NA of the laser beam of the laser treatment system differ by a factor of <2.

13. Arrangement according to Claim 1, **characterized in that** an NA of the OCDR system and an NA of the laser beam of the laser treatment system differ by a factor of <2.

14. Arrangement according to Claim 1, **characterized in that** wavelengths of the OCDR system and of the laser beam of the laser treatment system deviate by no more than 10% from one another.

15. Arrangement according to Claim 1, **characterized in that** the control unit is designed such that the deviation of the approximate determination of the assumable focus position PF of the laser beam is less than 2 Rayleigh lengths of the laser focus, in particular less than 1 Rayleigh length or particularly preferably less than 0.5 of the Rayleigh length.

## Revendications

1. Agencement pour le réétalonnage du foyer d'un système ophtalmologique pour le traitement au laser intraoculaire, constitué par une unité laser de traitement, une unité d'affichage et un système optique pour la focalisation et la superposition des faisceaux ainsi qu'un système d'OCDR (réflectométrie à domaine de cohérence optique) et une unité de commande, **caractérisé en ce que** le système de traitement au laser est réalisé de telle sorte que la distance A par rapport à l'œil peut être modifiée et un faisceau laser de visée peut être focalisé sur au moins une structure cible ZS₁ dans l'œil à traiter, **en ce que** l'unité de commande est réalisée pour déterminer une distance A₁ à partir de la position d'une structure de référence PRS sélectionnée, ainsi que la position de la structure cible PZS₁ dans le profil de signal d'OCDR, à chaque fois par rapport à un plan de référence RE et **en ce que** l'unité de commande est en outre réalisée pour évaluer approximativement, pour des valeurs pouvant être sélectionnées de manière quelconque d'une modification de distance ΔA du système de traitement au laser par rapport à l'œil, la position de focalisation PF à adopter à chaque fois du faisceau laser du système de traitement laser dans le profil de signal d'OCDR à l'aide des paramètres A₁ et PZS₁ .

2. Agencement selon la revendication 1, **caractérisé en ce que** l'unité de commande est réalisée pour utiliser les valeurs A₁ et PZS₁ trouvées, pour déterminer une fonction $PF \left(ΔA\right) = {f}_{{A}_{1} , PZ {S}_{1}} \left(ΔA\right)$ afin de calculer approximativement, pour des valeurs pouvant être sélectionnées de manière quelconque d'une modification de distance ΔA du système de traitement au laser par rapport à l'œil, la position de focalisation PF à adopter à chaque du faisceau laser fois du système de traitement au laser dans le profil de signal d'OCDR.

3. Agencement selon la revendication 1, **caractérisé en ce que** le système de traitement au laser est réalisé de telle sorte que le faisceau laser de visée peut être focalisé, en dehors d'être focalisé sur ZS₁, progressivement sur N-1 autres structures cibles ZS₂ ... ZS_{N} et **en ce que** l'unité de commande est réalisée pour déterminer, dans le profil de signal d'OCDR, pour celles-ci les positions respectives des structures cibles PZS₂ ... PZS_{N} et les modifications respectives ΔA₂ ... ΔA_{N} de la position de la structure de référence par rapport à sa position de départ PRS=A₁ lors de la focalisation sur la première structure cible ZS1 et pour ensuite déterminer, à partir des paramètres A_{1/}*ΔA*₂ .. *ΔA_{N},PZS*₁ ... *PZS_{N}*, une fonction *PF*(*ΔA*) - *f*_{A₁,*ΔA,... ΔAN,DZSn ... DZSN*}(*ΔA*) à partir de laquelle on peut alors calculer approximativement, pour des valeurs pouvant être sélectionnées de manière quelconque d'une modification de distance ΔA du système de traitement au laser par rapport à l'œil, la position de focalisation PF à adopter à chaque fois du faisceau laser du système de traitement au laser dans le profil de signal d'OCDR.

4. Agencement selon la revendication 1, **caractérisé en ce que** le système de traitement au laser est réalisé de telle sorte qu'on peut utiliser, comme faisceau laser de visée, le faisceau laser de traitement du système de traitement au laser présentant une énergie impulsionnelle réduite, qui ne peut pas déclencher de photodisruption.

5. Agencement selon la revendication 1, 2, 3 ou 4, **caractérisé en ce que** l'unité de commande est réalisée pour constater la détection d'une focalisation sur une structure cible par une constatation automatisée d'un ou de plusieurs des états suivants : 1) rétrodiffusion maximalisée du laser de visée par la structure cible 2) diamètre minimalisé de la distribution de la lumière du faisceau laser de visée sur la structure cible 3) état caractéristique ou modification caractéristique du signal d'OCDR de la structure cible.

6. Agencement selon la revendication 1, **caractérisé en ce que** le système de traitement au laser dispose de plusieurs lasers de visée qui se croisent au point de focalisation du laser de traitement du système de traitement au laser et qui servent à la focalisation sur une structure cible ZS dans l'œil à traiter, un système caméra étant présent, qui identifie la focalisation de lasers de visée sur la structure cible ZS par minimalisation de la distance des positions de laser de visée cible.

7. Agencement selon la revendication 1, **caractérisé en ce que** le système de traitement au laser est réalisé pour générer lui-même des structures cibles ZS dans l'œil.

8. Agencement selon la revendication 4, **caractérisé en ce que**
le système de traitement au laser est réalisé pour générer une structure cible ZS dans l'œil par une impulsion ou une modulation du laser de visée, qui est une modification dans l'œil, qui provoque une modification de signal dans l'OCDR ou une rétrodiffusion modifiée ou une modification de phase ou de granularité dans le signal d'OCDR.

9. Agencement selon la revendication 1, **caractérisé en ce que** le système de traitement au laser est réalisé pour générer une structure cible ZS dans l'œil qui est temporaire ou, selon le cas, modifiable, par exemple une bulle de gaz générée par au moins un coup de laser ou une structure de granularité modifiée temporairement suite à une modification de température dans l'OCDR.

10. Agencement selon la revendication 1, **caractérisé en ce que** l'unité de commande est réalisée pour exploiter la structure cible ZS générée par un coup de laser du faisceau laser du système de traitement au laser, non seulement pour la détermination des couches de focalisation, mais également pour le titrage de la puissance du laser.

11. Agencement selon la revendication 1, **caractérisé en ce que** le système de couches de réflexion diélectriques est réalisé de telle sorte que les faisceaux laser de visée sont réfléchis à une longueur d'onde entre 400 nm ... 1050 nm et le faisceau laser de traitement du système de traitement au laser est principalement transmis à des longueurs d'onde > 1050 nm.

12. Agencement selon la revendication 1, **caractérisé en ce que** le système d'affichage et le faisceau laser du système de traitement au laser présentent des ON qui se distinguent d'un facteur <2.

13. Agencement selon la revendication 1, **caractérisé en ce que** le système d'OCDR et le faisceau laser du système de traitement au laser présentent des ON qui se distinguent d'un facteur <2.

14. Agencement selon la revendication 1, **caractérisé en ce que** le système d'OCDR et le faisceau laser du système de traitement au laser présentent des longueurs d'ondes qui s'écartent de maximum 10% les unes des autres.

15. Agencement selon la revendication 1, **caractérisé en ce que** l'unité de commande est réalisée de telle sorte que l'écart de la détermination approximative de la position de focalisation PF à adopter du faisceau laser est inférieur à 2 longueurs de Rayleigh de la focalisation du laser, en particulier inférieur à 1 longueur de Rayleigh ou de manière particulièrement préférée inférieur à 0,5 de la longueur de Rayleigh.
